# EUROPÄISCHE PATENTSCHRIFT

(11) **EP 3 570 902 B1**
(45) Veröffentlichungstag und Bekanntmachung des Hinweises auf die Patenterteilung: **13.12.2023**
(21) Anmeldenummer: 18700849.5
(22) Anmeldetag: 03.01.2018
(51) Int. Cl.: A61M 1/14, A61M 1/16

(54) **DIALYSATKONZENTRATION-MESSSENSORDIAGNOSE**
DIALYSATE CONCENTRATION MEASUREMENT SENSOR DIAGNOSIS
DIAGNOSTIC PAR CAPTEUR DE MESURE DE LA CONCENTRATION DE DIALYSAT

(30) Priorität: 20.01.2017 DE 102017000495
(43) Veröffentlichungstag der Anmeldung: 27.11.2019
(73) Patentinhaber: Fresenius Medical Care Deutschland GmbH, 61352 Bad Homburg (DE)
(72) Erfinder: GAGEL, Alfred, 96123 Litzendorf (DE); STÄBLEIN, Tilman, 97074 Würzburg (DE)
(74) Vertreter: Rau, Jenspeter
(86) Internationale Anmeldenummer: PCT/EP2018/050104
(87) Internationale Veröffentlichungsnummer: WO 2018/134045

(56) Entgegenhaltungen:
- EP-A1- 2 767 296
- WO-A1-2016/125902

## Beschreibung

### TECHNISCHES GEBIET

Die vorliegende Erfindung betrifft ein Diagnoseverfahren für ein Dialyseflüssigkeitszubereitungsgerät und ein persönliches Dialysegerät, die Wasser, eine A-Flüssigkeit und eine B-Flüssigkeit jeweils in vorgegebenen Anteilen mischen, um eine Dialyseflüssigkeit herzustellen, und die Konzentration der zubereiteten Dialyseflüssigkeit durch Konzentrationsmessmittel messen.

### HINTERGRUND

Herkömmlich ist ein Dialyseflüssigkeitszubereitungsgerät bekannt, das einen Wasserzuleitungskanal, der Wasser zuleitet, einen A-Flüssigkeitskanal, der mit dem Wasserzuleitungskanal verbunden ist und eine A-Flüssigkeit zuleitet, die Natriumchlorid als einen Hauptinhaltsstoff enthält, und einen B-Flüssigkeitskanal, der an den Wasserzuleitungskanal angeschlossen ist und eine B-Flüssigkeit zuleitet, die aus einer wässrigen Natriumbicarbonatlösung besteht, unfasst, um eine Dialyseflüssigkeit zuzubereiten, die in einem Dialysegerät verwendet wird.

Ein solches Dialyseflüssigkeitszubereitungsgerät enthält Konzentrationsmessmittel, die mit einer Detektionseinheit zur Messung der elektrischen Leitfähigkeit stromabwärts der Anschlusspositionen des A-Flüssigkeitskanals und des B-Flüssigkeitskanals am Wasserzuleitungskanal bereitgestellt sind, um zu beurteilen, ob die zubereitete Dialyseflüssigkeit der erwarteten Zusammensetzung entspricht. Die Japanische Patentoffenlegungsgeschrift Nr. 6-142191 beschreibt ein Dialyseflüssigkeitszubereitungsgerät, das zur Erhöhung der Sicherheit bei einer Dialysebehandlung vor der Zubereitung der Dialyseflüssigkeit zunächst das Verhalten von Ein/Aus-Ventilen und dergleichen prüft, die darin vorgesehen sind.

EP 2767296 A1 beschreibt ein Verfahren zur Bewertung der Genauigkeit eines Leitfähigkeitssensors durch Zuleiten des wässrigen Natriumbicarbonatlösungskonzentrats zum Wasserzuleitungskanal und Messen des Leitfähigkeitswert durch dessen Vergleich mit einem im Voraus eingestellten Wert. WO2016125902 beschreibt eine Vorrichtung und ein Verfahren zur Verbesserung der Messgenauigkeit einer Konzentrationsmessvorrichtung eingesetzt zur Herstellung einer therapeutischen Flüssigkeit, ohne die Belastung für das Gesundheitspersonal während einer Vorbereitungsphase für eine Behandlung zu erhöhen, und ohne die Notwendigkeit, weitere Sensor hinzuzufügen. Dazu werden eine Elektrolytzufuhreinrichtung und eine Kalibriereinrichtung vorgesehen, die einen Elektrolyten mit bekannter Konzentration in einem den Mischkreislauf zuführt, und die Leitfähigkeitssensoren am Mischkreislauf kalibriert.

JP4458346 beschreibt eines medizinischen Geräts, das in der Lage ist, kostengünstig ein Dialysat in großen Mengen zu produzieren, ohne dass die mühsame Reinigung und Sterilisation des Geräts in einem Notfall- / Intensivbehandlungsbereich erforderlich ist. Ein typischer Aufbau der medizinischen Vorrichtung ist die medizinische Vorrichtung mit einer Zuführeinrichtung für eine unverdünnte Lösung A zum Zuführen der unverdünnten Lösung A, die kein Natriumbicarbonat zur Herstellung des Dialysats enthält, und einer Zuführeinrichtung für eine unverdünnte Lösung B zum Zuführen des unverdünnten B Natriumbicarbonat enthaltende Lösung zur Herstellung des Dialysats, eine Wasserversorgungseinrichtung zur Versorgung des Wassers zur Herstellung des Dialysats, eine Mischeinrichtung zum Mischen der unverdünnten Lösung A und Bund des Wassers zur Herstellung des Dialysats, und einer Speichereinrichtung zum Speichern das Dialysats.

### KURZDARSTELLUNG DER ERFINDUNG

### Problemstellung

EP 2767296 A1 beschreibt den Vorteil einer Verwendung der Bicarbonatlösung zur Diagnose, da sie immer dieselbe Konzentration hat, während die anderen Flüssigkeiten, die mit der Bicarbonatflüssigkeit gemischt werden, um die Dialyseflüssigkeit zu erzeugen, unterschiedliche Konzentrationen aufweisen können.

Die Erfinder haben jedoch erkannt, dass dies nur unter besonderen Umständen zutrifft, und haben daher beabischtigt, ein Verfahren und ein Gerät zu entwickeln, das eine Bewertung der Genauigkeit eines Leitfähigkeitssensors ermöglicht, die von dem verwendeten und allgemein anwendbaren System unabhängiger ist.

Beim Vorbereiten der Geräte für eine Hämodialyse oder eine Variante davon, wie Hämodiafiltration, hochvolumige Hämodiafiltration oder jede andere chronische oder akute extrakorporale Blutbehandlung, die Dialysat einsetzt, können die A- und die B-Konzentrate bereitgestellt werden als Lösungen, beispielsweise in Kanistern, oder als Trockenkonzentrate, beispielsweise in flexiblen Beuteln oder formstabilen Kartuschen, welchen Wasser zugeleitet wird, um ein flüssiges Konzentrat zu erzeugen, das in den Wasserzuleitungskanal dosiert wird, um ein Dialysat zu erzeugen.

Falls gebrauchsfertige Lösungen verwendet werden, sind beide Konzentrate normalerweise vollständig aufgelöst und die Konzentration der gelösten Ionen, und daher die Leitfähigkeit, ist vor dem Aufrüsten der Maschine bekannt.

Die Situation ist anders, wenn die Konzentrate als Trockenkonzentrate bereitgestellt werden. Für das B-Konzentrat, das aus einem einzigen Salz besteht, das normalerweise Natriumbicarbonat ist, wird eine gesättigte Lösung erzeugt, wobei festes Natriumbicarbonat im Behälter verbleibt, wo die Lösung erzeugt wird.

Die Löslichkeit von Bicarbonatsalzen ist jedoch temperaturabhängig und daher muss die Temperatur der Lösung bekannt sein, um die vorhergesagte Leitfähigkeit der Lösung für eine Diagnose der Präzision des Leitfähigkeitssensors zu beurteilen. Daher ist ein zusätzlicher Aufwand erforderlich, um die vorhergesagte Leitfähigkeit einer gesättigten Bicarbonatlösung zu bestimmen, der insbesondere die Bereitstellung eines Temperatursensors zur Messung der Temperatur im Behälter und eines Verarbeitungsmittels zum Berechnen der Leitfähigkeit für die gemessene Temperatur beinhaltet.

Die Zielsetzung ist, ein Leitfähigkeitssensor-Diagnoseverfahren und ein Gerät zur Behebung des obengenannten Problems bereitzustellen.

### Mittel zur Problemlösung

Die obengenannten Probleme werden durch ein Diagnoseverfahren und ein Dialyseflüssigkeitszubereitungsgerät der unabhängigen Ansprüche gelöst. Die abhängigen Ansprüche beschreiben Ausführungsformen des Verfahrens und des Geräts der unabhängigen Ansprüche. Die Erfinder haben erkannt, dass das A-Konzentrat normalerweise mindestens zwei verschiedenen Komponenten, beispielsweise Salze, aufweisen und daher das Konzentrat in Gebrauch vollkommen aufgelöst bereitgestellt werden muss, um zu gewährleisten, dass die Konzentration und insbesondere die relative Konzentration der Komponenten in der Lösung festgelegt ist. Das beudetet, dass die Konzentration, inbesonders die relative Konzentration sich nicht verändert wenn sich mehr oder weniger Flüssigkeit in dem Behälter, der die Komponenten enthält, befindet und unabhängig von der Temperatur. Dies gilt für beide Fälle, die Bereitstellung als Lösung oder die Bereitstellung als Trockenkonzentrat, das an der Maschine aufzulösen ist.

Das Diagnoseverfahren kann in einem Dialyseflüssigkeitszubereitungsgerät angewendet werden, das einen Wasserzuleitungskanal zum Zuleiten von Wasser, einen A-Flüssigkeitskanal zum Zuleiten einer A-Flüssigkeit, die Natriumchlorid als einen Hauptinhaltsstoff gemeinsam mit mindestens einem anderen Inhaltsstoff enthält, wobei der A-Flüssigkeitskanal an den Wasserzuleitungskanal angeschlossen ist, einen B-Flüssigkeitskanal zum Zuleiten einer B-Flüssigkeit, die aus einer wässrigen Natriumbicarbonatlösung besteht, wobei der B-Flüssigkeitskanal an den Wasserzuleitungskanal angeschlossen ist, und Konzentrationsmessmitten, die mit einer Detektionseinheit stromabwärts der Anschlusspositionen des A-Flüssigkeitskanals und des B-Flüssigkeitskanals an den Wasserzuleitungskanal bereitgestellt sind, aufweist. Das Diagnoseverfahren kann ein Mischen des Wassers, der A-Flüssigkeit und der B-Flüssigkeit jeweils in vorgegebenen Anteilen, um eine Dialyseflüssigkeit zuzubereiten, und ein Messen einer Konzentration der zubereiteten Dialyseflüssigkeit mit dem Konzentrationsmessmittel, und, vor dem Zubereiten der Dialyseflüssigkeit, ein Zuleiten der A-Flüssigkeit aus dem A-Flüssigkeitskanal in den Wasserzuleitungskanal, so dass nur die A-Flüssigkeit in den Wasserzuleitungskanal fließt, und ein Diagnostizieren einer Genauigkeit des Konzentrationsmessmittels durch Messen einer elektrischen Leitfähigkeit der A-Flüssigkeit, unverdünnt oder verdünnten, mit dem Konzentrationsmessmittel und ein Vergleichen der gemessenen elektrischen Leitfähigkeit der A-Flüssigkeit bzw. der verdünnten A-Flüssigkeit mit einem zuvor eingestellten Wert umfassen.

Das Diagnoseverfahren kann mittels A-Flüssigkeit in einer unverdünnten oder in einer verdünnten Form durchgeführt werden. Das bedeutet, dass entweder nur A-Flüssigkeit zu dem Konzentrationsmessmittel transportiert wird oder die A-Flüssigkeit mit einer bekannten Menge an Wasser, beispielsweise aus dem Wasserzuleitungskanal, verdünnt wird. In beiden Fällen ist der zuvor eingestellte Wert ein Leitfähigkeitswert, der für die jeweilige Zusammensetzung der A-Flüssigkeit in verdünnter oder unverdünnter Form erwartet wird. Wenn die gemessene Leitfähigkeit, jedoch, nicht gemessen wird, ist das ein Hinweis darauf, dass eine der Komponenten der Konzentrationsmessmittel nicht richtig funktioniert.

Das Diagnoseverfahren für das Dialyseflüssigkeitszubereitungsgerät kann beim Messen der elektrischen Leitfähigkeit der A-Flüssigkeit ein Messen einer Zeit, nachdem eine A-Flüssigkeitspumpe begonnen hat, eine Flüssigkeitsbeschickung der A-Flüssigkeit zu starten, und bevor das Konzentrationsmessmittel eine Konzentration der A-Flüssigkeit misst, wobei die A-Flüssigkeitspumpe am A-Flüssigkeitskanal bereitgestellt ist und die A-Flüssigkeit in den Wasserzuleitungskanal leitet, und somit ein Diagnostizieren einer Flüssigkeitsbeschickungsgenauigkeit der A-Flüssigkeitspumpe umfassen.

Dieser Test erlaubt es, einen potentielle Fehler auszuschließen, der darin liegt, dass die A-Flüssigkeitspumpe eine Fehlfunktion hat, welche zu einer Abweichung der gemessenen Leitfähigkeit vom zuvor eingestellten Wert, und erlaubt es daher, die Wahrscheinlichkeit zu erhöhen, dass die Abweichung von einer Fehlfunktion des Sensors des Konzentrationsmessmittel herrührt.

Zur Herstellung der verdünnten A-Flüssigkeit, kann in dem Diagnosverfahren für das Dialyseflüssigkeitszubereitungsgerät das Wasser und die A-Flüssigkeit gemischt werden, um eine Zubereitungsflüssigkeit der A-Flüssigkeit durch Einströmen des Wassers in den Wasserzuleitungskanal zuzubereiten.

Das Diagnoseverfahren für das Dialyseflüssigkeitszubereitungsgerät kann ein Anzeigen einer Nachricht auf einer Anzeige des Dialyseflüssigkeitszubereitungsgeräts umfassen, dass der Benutzer prüfen soll, ob eine Art von A-Flüssigkeit, die als Eingabeparameter zum Bestimmen des zuvor eingestellten Werts verwendet wird, einer Art von A-Flüssigkeit der A-Flüssigkeit entspricht. Diese Nachricht kann auf Grund des Ergebnisses des Vergleichs der gemessenen elektrischen Leitfähigkeit der A-Flüssigkeit oder der verdünnten A-Flüssigkeit mit dem zuvor ermittelten Wert, insbesonders wenn eine Abweichung zwischen der gemessenen elektrischen Leitfähigkeit und dem zuvor ermittelten Wert einen Grenzwert überschreitet, angezeigt werden.

Dieser Test erlaubt es, einen weiteren potentielle Fehler auszuschließen, der darin liegt, dass eine Nichtübereinstimmung zwischen der A-Flüssigkeit, die mit dem Dialyseflüssigkeitszubereitungsgerät verbunden ist und dem ermittelten Wert in dem Gerät vorliegt, welche zu einer Abweichung der gemessenen Leitfähigkeit vom zuvor eingestellten Wert, und erlaubt es daher, die Wahrscheinlichkeit zu erhöhen, dass die Abweichung von einer Fehlfunktion des Sensors des Konzentrationsmessmittel herrührt.

Das Diagnoseverfahren für das Dialyseflüssigkeitszubereitungsgerät kann des Weiteren ein Anzeigen einer Fehlernachricht auf der Anzeige des Dialyseflüssigkeitszubereitungsgeräts umfassen, die eine Fehlfunktion des Dialyseflüssigkeitszubereitungsgeräts anzeigt, wenn eine Art von A-Flüssigkeit, die als Eingabeparameter zum Bestimmen des zuvor eingestellten Werts verwendet wird, einer Art von A-Flüssigkeit der A-Flüssigkeit entspricht. Diese Nachricht kann auf Grund des Ergebnisses des Vergleichs der gemessenen elektrischen Leitfähigkeit der A-Flüssigkeit oder der verdünnten A-Flüssigkeit mit dem zuvor ermittelten Wert, insbesonders wenn eine Abweichung zwischen der gemessenen elektrischen Leitfähigkeit und dem zuvor ermittelten Wert einen Grenzwert überschreitet, angezeigt werden.

Das Diagnoseverfahren für das Dialyseflüssigkeitszubereitungsgerät kann ein Bereitstellen eines Benutzerinteraktionsmittels, das ein Touchscreen oder ein Bedienungsfeld oder eine Taste oder jede andere bekannte Art von Benutzerinteraktionsvorrichtung sein kann, um zu bestätigen, dass die Art von A-Flüssigkeit, die als Eingabeparameter zum Bestimmen des zuvor eingestellten Werts verwendet wird, der Art von A-Flüssigkeit der A-Flüssigkeit entspricht, und ein Anzeigen einer Warnnachricht auf der Anzeige des Dialyseflüssigkeitszubereitungsgeräts, beispielsweise dass der Benutzer den Wartungsdienst benachrichtigen soll, wenn das Benutzerinteraktionsmittel vom Benutzer aktiviert wurde. Diese Interaktionsmittel kann ein Aktivierungsknopf sein, der auf Grund des Ergebnisses des Vergleichs der gemessenen elektrischen Leitfähigkeit der A-Flüssigkeit oder der verdünnten A-Flüssigkeit mit dem zuvor ermittelten Wert, insbesonders wenn eine Abweichung zwischen der gemessenen elektrischen Leitfähigkeit und dem zuvor ermittelten Wert einen Grenzwert überschreitet, angezeigt wird

Das Diagnoseverfahren für das Dialyseflüssigkeitszubereitungsgerät kann umfassen, dass der zuvor eingestellte Wert ein Wert ist, der auf einer weiteren elektrischen Leitfähigkeit beruht, die durch ein weiteres Detektionsmittel eines weiteren Konzentrationsmessmittels gemessen wird, das im A-Flüssigkeitskanal oder Wasserzuleitungskanal stromabwärts des Anschlusses des A-Flüssigkeitskanals an den Wasserzuleitungskanal positioniert ist.

Indem der zuvor ermittelte Wert auf diese Art und Weise bestimmt wird, ist es möglich, unabhängig von der Eingabe eines Nutzers oder irgendeines anderen Datentransfers zu sein. Ferner ist das Risiko eines Fehlers auf Grund eines Pumpfehlers reduziert, da es dieselbe Flüssigkeit ist, die beide Konzentrationsmessmittelssensoren passiert.

Ein Dialyseflüssigkeitszubereitungsgerät gemäß der Erfindung umfasst einen Wasserzuleitungskanal zum Leiten von Wasser, eine Wasserbeschickungspumpe, die am Wasserzuleitungskanal bereitgestellt ist, einen A-Flüssigkeitskanal zum Zuleiten einer A-Flüssigkeit, die Natriumchlorid als einen Hauptinhaltsstoff gemeinsam mit mindestens einem anderen Inhaltsstoff enthält, wobei der A-Flüssigkeitskanal an den Wasserzuleitungskanal angeschlossen ist, eine A-Flüssigkeitspumpe, die am A-Flüssigkeitskanal bereitgestellt ist, einen B-Flüssigkeitskanal zum Zuleiten einer B-Flüssigkeit, die aus einer wässrigen Natriumbicarbonatlösung besteht, wobei der B-Flüssigkeitskanal an den Wasserzuleitungskanal angeschlossen ist, eine B-Flüssigkeitspumpe, die am B-Flüssigkeitskanal bereitgestellt ist, ein Konzentrationsmessmittel, das mit einer Detektionseinheit stromabwärts der Anschlusspositionen des A-Flüssigkeitskanals und des B-Flüssigkeitskanals am Wasserzuleitungskanal bereitgestellt ist, und ein Steuermittel zum Steuern von Betätigungen der Pumpen. Das Steuermittel des Dialyseflüssigkeitszubereitungsgeräts ist konfiguriert, ein Mischen des Wassers, der A-Flüssigkeit und der B-Flüssigkeit in vorgegebenen Anteilen zu steuern, um eine Dialyseflüssigkeit zuzubereiten. Ferner ist das Steuermittel mit einer Diagnoseeinheit zum Diagnostizieren der Genauigkeit des Konzentrationsmessmittels durch Betätigen der A-Flüssigkeitspumpe konfiguriert, um eine Flüssigkeitsbeschickung nur der A-Flüssigkeit in den Wasserzuleitungskanal auszuführen, insbesondere, ohne eine Flüssigkeitsbeschickung mit der B-Flüssigkeitspumpe durchzuführen, wobei die Diagnoseeinheit konfiguriert ist, die Genaugkeit des Konzentrationsmessmittels durch einen Vergleich einer elektrischen Leitfähigkeit der A-Flüssigkeit, unverdünnt oder verdünnt, die durch die Detektionseinheit des Konzentrationsmessmittels gemessen wird, mit einem zuvor eingestellten Wert durchzuführen.

Das Dialyseflüssigkeitszubereitungsgerät kann ferner konfiguriert sein, beim Messen der elektrischen Leitfähigkeit der A-Flüssigkeit eine Zeit, nachdem eine A-Flüssigkeitspumpe begonnen hat, eine Flüssigkeitsbeschickung der A-Flüssigkeit zu starten, und bevor das Konzentrationsmessmittel eine Konzentration der A-Flüssigkeit misst, zu messen und dadurch eine Flüssigkeitsbeschickungsgenauigkeit der B-Flüssigkeitspumpe zu diagnostizieren.

Das Dialyseflüssigkeitszubereitungsgerät kann ferner konfiguriert sein, zum Herstellen der verdünnten A-Flüssigkeit, das Wasser und die A-Flüssigkeit zu mischen, um eine Zubereitungsflüssigkeit der A-Flüssigkeit zuzubereiten

Das Dialyseflüssigkeitszubereitungsgerät kann ferner eine Anzeige umfassen. Die Anzeige kann ein Bildschirm, ein Berührungsschirm (Touch Screen), ein Anzeigelicht oder jede andere Vorrichtung sein, die einem Benutzer Informationen liefert.

Das Dialyseflüssigkeitszubereitungsgerät kann ferner, zum Anzeigen einer Nachricht auf der Anzeige konfiguriert sein, dass der Benutzer prüfen soll, ob eine Art von A-Flüssigkeit, die als Eingabeparameter zum Bestimmen des zuvor eingestellten Werts verwendet wird, einer Art von A-Flüssigkeit der A-Flüssigkeit entspricht. Diese Nachricht kann auf Grund des Ergebnisses des Vergleichs der gemessenen elektrischen Leitfähigkeit der A-Flüssigkeit oder der verdünnten A-Flüssigkeit mit dem zuvor ermittelten Wert, insbesonders wenn eine Abweichung zwischen der gemessenen elektrischen Leitfähigkeit und dem zuvor ermittelten Wert einen Grenzwert überschreitet, angezeigt werden.

Das Dialyseflüssigkeitszubereitungsgerät kann fernerzum Anzeigen einer Fehlernachricht auf der Anzeige konfiguriert sein, die eine Fehlfunktion des Dialyseflüssigkeitszubereitungsgeräts angibt, wenn eine Art von A-Flüssigkeit, die als Eingabeparameter zum Bestimmen des zuvor eingestellten Werts verwendet wird, einer Art von A-Flüssigkeit der A-Flüssigkeit entspricht. Diese Nachricht kann auf Grund des Ergebnisses des Vergleichs der gemessenen elektrischen Leitfähigkeit der A-Flüssigkeit oder der verdünnten A-Flüssigkeit mit dem zuvor ermittelten Wert, insbesonders wenn eine Abweichung zwischen der gemessenen elektrischen Leitfähigkeit und dem zuvor ermittelten Wert einen Grenzwert überschreitet, angezeigt werden.

Das Dialyseflüssigkeitszubereitungsgerät kann ferner ein Benutzerinteraktionsmittel umfassen.

Das Dialyseflüssigkeitszubereitungsgerät kann konfiguriert sein bei Aktivierung des Benutzerinteraktionsmittels, die bestätigt, dass die Art von A-Flüssigkeit, die als Eingabeparameter zum Bestimmen des zuvor eingestellten Werts verwendet wird, der Art von A-Flüssigkeit der A-Flüssigkeit entspricht, zum Anzeigen einer Fehlernachricht auf der Anzeige der Dialyseflüssigkeitszubereitung, die eine Fehlfunktion der Dialyseflüssigkeitszubereitung angibt.

Das Dialyseflüssigkeitszubereitungsgerät kann ferner ein weiteres Detektionsmittel eines weiteren Konzentrationsmessmittels umfassen, das im A-Flüssigkeitskanal oder Wasserzuleitungskanal stromabwärts des Anschlusses des A-Flüssigkeitskanals an den Wasserzuleitungskanal angordnet ist. Das Dialyseflüssigkeitszubereitungsgerät kann ferner zum Verwenden eines Wertes, der auf einer weiteren elektrischen Leitfähigkeit beruht, die durch das weitere Detektionsmittel gemessen wird, als zuvor eingestellten Wert konfiguriert sein.

In allen Ausführungsformen ist hier die Verwendung des Ausdrucks "kann sein" oder "kann haben" und so weiter soll eine beispielhafte Ausführungsform gemäß der vorliegenden Erfindung darstellen.

Ausführungsformen des Dialyseflüssigkeitszubereitungsverfahrens und des Dialyseflüssigkeitszubereitungsgeräts werden unter Bezugnahme auf die Figuren beschrieben.
Figur 1 zeigt eine schematische Konfigurationszeichnung eines Dialyseflüssigkeitszubereitungsgeräts gemäß einer ersten Ausführungsform,
Figur 2 zeigt eine schematische Konfigurationszeichnung eines persönlichen Dialysegeräts gemäß einer zweiten Ausführungsform, und
Figur 3 zeigt ein schematisches Ablaufdiagramm des erfindungsgemäßen Diagnoseverfahrens gemäß einer Ausführungsform.

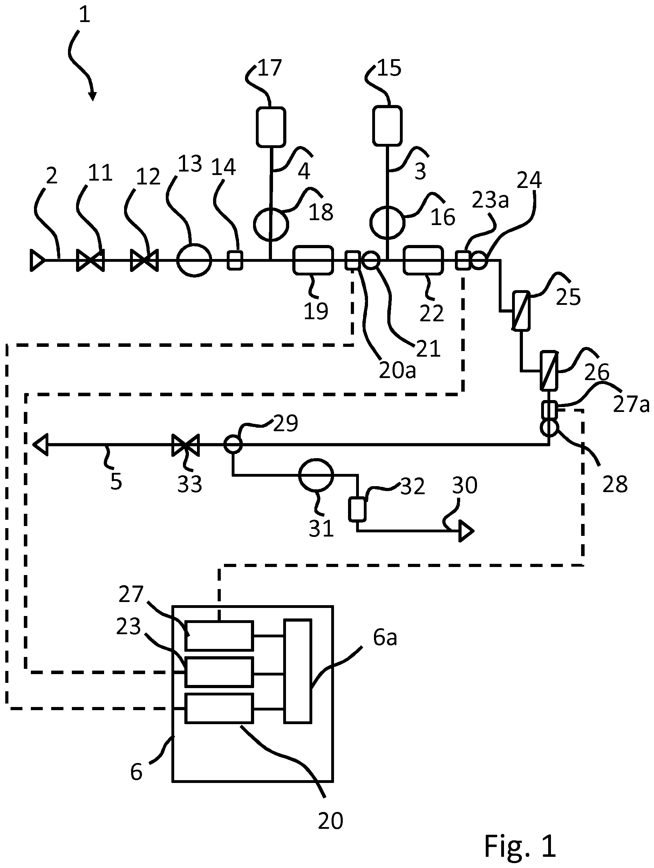

In der Folge werden Ausführungsformen, die in den Zeichnungen dargestellt sind, beschrieben. Figur 1 zeigt ein Dialyseflüssigkeitszubereitungsgerät 1, das in Spitälern und dergleichen installiert werden kann. Dieses Dialyseflüssigkeitszubereitungsgerät 1 kann eine vorbereitete Dialyseflüssigkeit zu einer Mehrzahl von Dialysemonitorgeräten, nicht gezeigt in der Figur leiten, als ein sogenanntes Dialyseflüssigkeitsbeschickungsgerät für mehrere Personen.

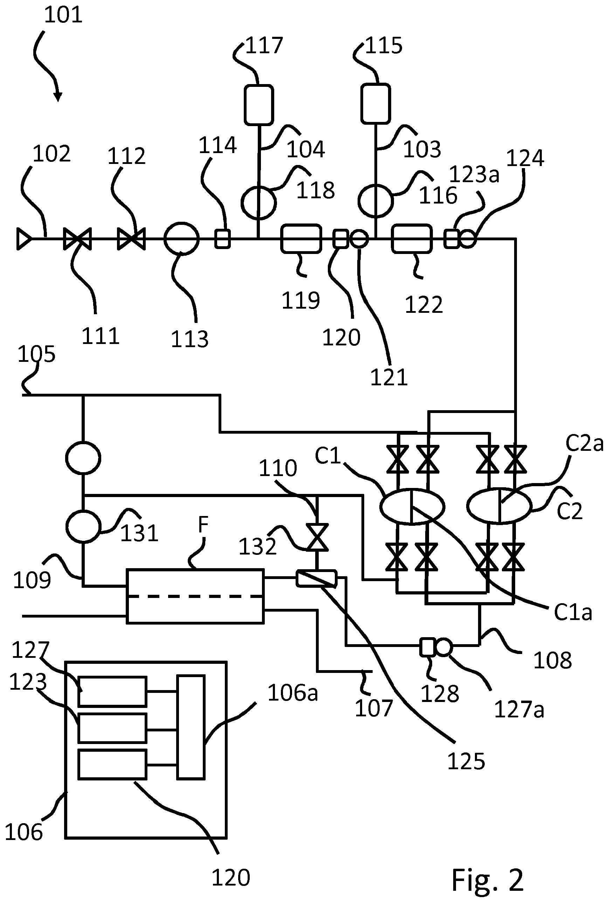

Figur 2, die eine zweite Ausführungsform zeigt, stellt eine Konfigurationszeichnung eines sogenannten persönlichen Dialysegeräts 101 dar. Dieses persönliche Dialysegerät 101 hat auch eine Funktion wie das Diagnoseflüssigkeitszubereitungsgerät 1, das in Figur 1 dargestellt ist, und kann die Diagnoseflüssigkeit zubereiten. Daher kann das Dialyseflüssigkeitszubereitungsgerät 1, wie in der Folge beschrieben, in das persönliche Dialysegerät 101 integriert sein.

Dementsprechend sind die Ausführungsformen des Dialyseflüssigkeitszubereitungsgeräts 1, wie in der Folge beschrieben, auch Ausführungsformen des persönlichen Dialysegeräts 101, wenn dieses in das persönliche Dialysegerät 101 integriert ist.

Zur Hervorhebung der Ähnlichkeit haben die entsprechenden Elemente im Dialyseflüssigkeitszubereitungsgerät 1 und im persönlichen Dialysegerät 101 die folgenden Bezugszeichen:
1: Dialyseflüssigkeitszubereitungsgerät - 101: Persönliches Dialysegerät
2: Wasserzuleitungskanal - 102
3: A-Flüssigkeitskanal - 103
4: B-Flüssigkeitskanal - 104
5: Abfallflüssigkeitskanal - 105
6: Steuermittel - 106
6a: Diagnoseeinheit - 106a
12: Stellventil - 112
13: Wasserpumpe - 113
14: Durchflussmesser - 114
15: A-Flüssigkeitstank - 115
16: A-Flüssigkeitspumpe - 116
17: B-Flüssigkeitstank - 117
18: B-Flüssigkeitspumpe - 118
19: Erster Puffertank - 119
20: Erstes Konzentrationsmessmittel - 120
20a: Erste Detektionseinheit - 120a
21: Temperatursensor - 121
22: Zweiter Puffertank - 122
23: Zweites Konzentrationsmessmittel - 123
23a: Zweite Detektionseinheit - 123a
24: Temperatursensor - 124
25: Erstes Filter - 125
26: Zweites Filter
27: Drittes Konzentrationsmessmittel - 127
27a: Dritte Detektionseinheit - 127a
28: Temperatursensor - 128
29: Anschlussstück
30: Flüssigkeitsbeschickungskanal - 108
31. Flüssigkeitsbeschickungspumpe - C in Kombination mit 131
32: Tropfkammer
Ein/Aus-Ventil 132
33: Ein/Aus-Ventil

Das Dialyseflüssigkeitszubereitungsgerät 1 enthält einen Wasserzuleitungskanal 2, der an ein Wasserbeschickungsmittel zum Zuleiten von Wasser angeschlossen ist, das in der Figur 1 nicht dargestellt ist, einen A-Flüssigkeitskanal 3, der an den Wasserzuleitungskanal 2 angeschlossen ist und eine A-Flüssigkeit als unverdünnten flüssigen Inhaltsstoff der Dialyseflüssigkeit zuleitet, einen B-Flüssigkeitskanal 4, der an den Wasserzuleitungskanal 2 angeschlossen ist und eine B-Flüssigkeit zuleitet. Das Dialyseflüssigkeitszubereitungsgerät kann ferner einen Abfallflüssigkeitskanal 5 umfassen, der vom Wasserzuleitungskanal 2 wegführt und eine schlechte Dialyseflüssigkeit ableitet. Eine oder mehrere Pumpe(n) 13, 18, 16 des Dialyseflüssigkeitszubereitungsgeräts 1 können durch ein Steuermittel 6 gesteuert werden (entsprechende Steuerleitungen sind nicht dargestellt). Die Betätigung von Ein/Aus-Solenoidventilen kann auch durch das Steuermittel 6 gesteuert werden. Das Wasser kann durch den Wasserzuleitungskanal 2 geleitet werden. Wenn das Dialysezubereitungsgerät 1 Teil eines persönlichen Dialysegeräts 101 ist, können daher der Wasserzuleitungskanal 2 oder der Wasserzuleitungskanal 102 durch weitere Elemente des Dialyseflüssigkeitszubereitungsgeräts 1 geleitet werden, wie eine Entgasungskammer, eine Heizkammer, eine Lufttrennkammer (die Elemente sind nicht dargestellt).

"Unverdünnt" hat die Bedeutung, dass eine höhere Konzentration mindestens eines Inhaltsstoffs vorliegt als in der Dialyselösung, die während der Behandlung verwendet wird. Im Prinzip bezeichnet "unverdünnt" das Material in den Behältern 15, 17 im Vergleich zu diesem Material, wenn es mit Wasser aus dem Wasserzuleitungskanal 2 gemischt ist.

Der Wasserzuleitungskanal 2 kann an der Einlassseite an ein Wasserreinigungsmittels zum Erzeugen gereinigten Wassers (nicht dargestellt) angeschlossen sein. Das Wasserreinigungsmittel kann ein RO Wassergenerator sein. Der RO Wassergenerator kann ein Entionisierungsmittel sein, um Wasser mit sehr niedriger und/oder stabiler und/oder vorgegebener elektrischer Leitfähigkeit bereitzustellen.

Die A-Flüssigkeit als eine unverdünnte Flüssigkeit der Dialyseflüssigkeit weist Natriumchlorid als Inhaltsstoff auf und enthält neben Natriumchlorid auch mindestens einen anderen Inhaltsstoff. Der andere Inhaltsstoff kann mindestens ein Material oder mehrere oder alle Materialien der Gruppe sein, die Magnesiumchlorid, Kalziumchlorid, Kaliumchlorid, Zitronensäure, Glucose, Dextrose umfasst. Das Verhältnis kann unterschiedlich sein, abhängig von dem Patienten, für den die Verwendung bestimmt ist, und daher kann die Konzentration der A-Flüssigkeit selbst für die zuzubereitende Dialyseflüssigkeit variieren.

Andererseits besteht die B-Flüssigkeit als unverdünnte Flüssigkeitskomponente der Dialyseflüssigkeit aus einer wässrigen Lösung, die nur Natriumbicarbonat enthält.

Wie später ausführlich beschrieben wird, hat das Dialyseflüssigkeitszubereitungsgerät 1 eine Selbstkontrollfunktion, um das Geräte, die das Dialyseflüssigkeitszubereitungsgerät 1 aufweist, im Voraus zu diagnostizieren, bevor die Dialyseflüssigkeit zubereitet wird, und kann zwischen einem Diagnosemodus zum Durchführen der Selbstkontrolle und einem Zubereitungsmodus zum Durchführen der Zubereitung der Dialyseflüssigkeit umschalten.

Der B-Flüssigkeitskanal 4 kann stromaufwärts des A-Flüssigkeitskanals 3 am Wasserzuleitungskanal 2 angeschlossen sein. Stromaufwärts der Anschlussposition des B-Flüssigkeitskanals 4 können ein erstes Ein/Aus-Ventil 11 zum Öffnen und Schließen des Wasserzuleitungskanals 2, eine Wasserpumpe 13 zum Beschicken des Wassers bereitgestellt sein. Ferner können ein Stellventil 12 zum Einstellen der Durchflussrate des gereinigten Wassers im Wasserzuleitungskanal 2 und/oder ein Durchflussmesser 14 zum Messen der Durchflussrate des Wassers an der stromaufwärts liegenden Seite des Anschlusspunkts des B-Flüssigkeitskanals 4 und/oder des A-Flüssigkeitskanals 3 an den Wasserzuleitungskanal 2 bereitgestellt sein.

Das Stellventil 12, das ein Nadelventil sein kann, kann so gestaltet sein, dass es mittels Steuerung des Steuermittels 6 die Durchflussrate des Wassers einstellt, das die Wasserpumpe 13 zuleitet. Alternativ oder zusätzlich kann das Steuermittel die Durchflussrate des Wassers durch Anpassen der Pumpgeschwindigkeit der Pumpe 13 steuern. In diesem Fall kann das Stellventil 12 weggelassen sein. Der Durchflussmesser 14 kann die Durchflussrate messen und meldet die Einstellung der Durchflussrate durch das Stellventil 12 zurück. Der A-Flüssigkeitskanal 3 hat ein Ende, das an einen A-Flüssigkeitstank 15 angeschlossen ist, in dem die A-Flüssigkeit gepoolt ist, und eine A-Flüssigkeitspumpe 16, die die A-Flüssigkeit zuleitet, ist am A-Flüssigkeitskanal 3 bereitgestellt. Ähnlich ist der B-Flüssigkeitskanal 4 an einen B-Flüssigkeitstank 17 angeschlossen, in dem die B-Flüssigkeit gepoolt ist, und eine B-Flüssigkeitspumpe 18, die die B-Flüssigkeit zuleitet, ist am B-Flüssigkeitskanal 4 bereitgestellt.

Der A-Flüssigkeitstank 15 und/oder der B-Flüssigkeitstank 17 können formstabile Kartuschen oder flexible Beutel sein. Die festen Inhaltsstoffe der A-Flüssigkeit und/oder der B-Flüssigkeit können im Tank 15, 17 in einer festen Phase bereitgestellt sein, beispielsweise als Pulver, Granulat oder ein Gemisch davon, und das Dialyseflüssigkeitszubereitungsgerät 1 kann einen Flüssigkeitsanschluss vom Wasserzuleitungskanal 2 zum Zuleiten von Wasser zu mindestens einem oder beiden von A-Flüssigkeitstank 15 und B-Flüssigkeitstank 17 enthalten. Somit können die unverdünnten Flüssigkeiten der A-Flüssigkeit und der B-Flüssigkeit erzeugt werden, nachdem die das feste Material enthaltenden Behälter 17, 15 am Dialyseflüssigkeitszubereitungsgerät 1 angebracht wurden. Es ist klar, dass auch Aufschlämmungen oder eine andere Beschaffenheit zwischen festen und vollständigen Lösungen verwendet werden können.

Die A-Flüssigkeitspumpe 16 und die B-Flüssigkeitspumpe 18 können jeweils Volumenpumpen sein und Motoren enthalten, die in den Figuren nicht dargestellt sind. Durch Betreiben dieser Motoren bei vorgegebenen Drehzahlen ist es möglich, die A-Flüssigkeit und die B-Flüssigkeit bei vorgegebenen Durchflussraten, die im Steuermittel 6 eingestellt sind, in den Wasserzuleitungskanal 2 zu leiten.

Zwischen dem B-Flüssigkeitskanal 4 und dem A-Flüssigkeitskanal 3 können am Wasserzuleitungskanal 2 mindestens eines von einem ersten Puffertank 19 zum vorübergehenden Poolen von Flüssigkeit, eine Detektionseinheit 20a eines ersten Konzentrationsmessmittels 20 zum Messen der Konzentration der Flüssigkeit und ein erstes Temperaturmessmittel 21 zum Messen der Temperatur der Flüssigkeit bereitgestellt sein.

Stromabwärts des A-Flüssigkeitskanals 3 ist am Wasserzuleitungskanal 2 eine Detektionseinheit 23a eines zweiten Konzentrationsmessmittels 23 zum Messen der Konzentration der Flüssigkeit bereitgestellt. Ferner können stromabwärts des A-Flüssigkeitskanals 3 mindestens eines oder mehrere der folgenden Elemente bereitgestellt sein: ein zweiter Puffertank 22 zum vorübergehenden Poolen von Flüssigkeit, ein zweites Temperaturmessmittel 24 zum Messen der Temperatur der Flüssigkeit, ein erstes Filter 25 zum Reinigen der Dialyseflüssigkeit, eine Detektionseinheit 27a eines dritten Konzentrationsmessmittels 27 zum Messen der Konzentration der Flüssigkeit und ein drittes Temperaturmessmittel 28 zum Messen der Temperatur der Flüssigkeit. Vorzugsweise sind die Temperatursensoren 21, 24, 28 in unmittelbarer Nähe der Detektionseinheiten 20a, 23a, 27a gelegen, da der Ausgang des Konzentrationsmessmittels beruhend auf dem Ausgang des Temperaturmessmittels temperaturkompensiert sein kann. Dies kann ein besseres Vergleichsergebnis der gemessenen elektrischen Leitfähigkeit und des zuvor eingestellten Werts ermöglichen.

Ein zweiter Filter 26 zum Reinigen der Dialyseflüssigkeit kann stromabwärts des ersten Filters 25 bereitgestellt sein.

Der Dialyseflüssigkeitspfad als Verlängerung des Wasserströmungspfades 2, in dem in Betrieb das im Dialyseflüssigkeitszubereitungsgerät 1 erzeugte Dialysat fließt, kann an den Abfallflüssigkeitskanal 5 durch ein Anschlussstück 29 angeschlossen sein. Das Anschlussstück 29 kann stromabwärts an mindestens einer der obengenannten Detektionseinheiten 20a, 23a, 27a positioniert sein. Ein Ein/Aus-Ventil 33 kann am Abfallflüssigkeitskanal 5 nach dem Anschlussstück 29 bereitgestellt sein.

Für eine eigenständige Ausführungsform des Dialyseflüssigkeitszubereitungsgeräts 1 können ferner eine Flüssigkeitsbeschickungspumpe 31 zum Zuleiten der Dialyseflüssigkeit und eine Tropfkammer 32 an einem Flüssigkeitsbeschickungskanal 30, der an den Dialyseflüssigkeitspfad angeschlossen ist, bereitgestellt sein. Durch Aktivieren dieser Pumpe kann das Dialysat einem oder mehreren Dialyseüberwachungsgeräten bereitgestellt werden. Die Flüssigkeitstropfkammer 32 kann auch fehlen.

Für eine Ausführungsform des persönlichen Dialysegeräts 101 kann eine Flüssigkeitsbeschickungspumpe, die beispielsweise ein Bilanziersystem aufweisen kann, das Bilanzierkammern C1, C2 und eine Pumpe 131 enthält, bereitgestellt sein, um die Dialyseflüssigkeit zum Dialysator F zu leiten, wobei das Ausgleichssystem stromaufwärts des Filters 125 bereitgestellt sein kann. Die Art von Bilanziersystem ist nicht auf ein Bilanzkammersystem beschränkt. Alternativ kann das persönliche Dialysegerät 101 eine Förderpumpe und Abfallpumpe haben und das Bilanzieren erfolgt durch Steuerung der Ströme, die von den zwei Pumpen erzeugt werden.

Der erste und zweite Puffertank 19, 22 bilden Räume, in welchen der Durchmesser eines Teils des Wasserszuleitungskanals 2 vergrößert ist und Flüssigkeit gemischt wird. Das Mischen kann durch Rühren erfolgen. Im ersten Puffertank 19 werden die B-Flüssigkeit und das gereinigte Wasser gemischt, so dass eine Zubereitungsflüssigkeit der B-Flüssigkeit zubereitet wird. Im zweiten Puffertank 22 wird die A-Flüssigkeit mit der Zubereitungsflüssigkeit der B-Flüssigkeit gemischt, so dass die Dialyseflüssigkeit zubereitet wird. Das Verhältnis zwischen der B-Flüssigkeit und dem Wasser in der Zubereitungsflüssigkeit von B kann mit dem Verhältnis zwischen der B-Flüssigkeit und dem Wasser in der Dialyseflüssigkeit identisch sein, die durch das Dialyseflüssigkeitszubereitungsgerät 1 zubereitet wird. Die Konzentration der B-Flüssigkeit kann gemessen und im Steuermittel 6 registriert werden. Dasselbe gilt für die A-Flüssigkeit.

Ebenso kann in Bezug auf die Dialyseflüssigkeit die Konzentration zuvor gemessen und im Steuermittel 6 registriert werden. In Bezug auf diese Konzentration werden mehrere Arten registriert, abhängig von der zu verwendenden A-Flüssigkeit. Ebenso können weitere Leitfähigkeiten für verschiedene A-Flüssigkeiten und B-Flüssigkeiten und verschiedene Mischverhältnisse registriert werden. Registrierung umfasst auch ein Bereitstellen eines Algorithmus zum Berechnen der elektrischen Leitfähigkeit basierend auf der Komponente und ihrem Verhältnis, beispielsweise Wasser, A-Flüssigkeit und B-Flüssigkeit, wobei deren Registrierung auch die Berechnungsverfahren enthalten kann, bei welchen die Inhaltsstoffe für eine Art von A-Flüssigkeiten und auch Arten von B-Flüssigkeit mit ihren Konzentrationen im Voraus im Dialysatzubereitungsgerät 1 oder persönlichen Dialysegerät 101 gespeichert werden.

Falls vorhanden, können der erste und zweite Filter 25, 26 Endotoxine in der zubereiteten Dialyseflüssigkeit entfernen. Die Endotoxine werden durch die zwei Filter sicher entfernt.

Das erste bis dritte Konzentrationsmessmittel 20, 23, 27 sind Konzentrationsmesser, die die elektrischen Leitfähigkeiten der Flüssigkeiten, die von der ersten bis dritten Detektionseinheit 20a, 23a bzw. 27a gemessen werden, die Elektroden enthalten, in Konzentrationen der Flüssigkeiten umwandeln, und im Steuermittel 6 eingegliedert sind. Wenn jedoch der Ausdruck "Messen der Konzentration" verwendet wird, ist der Schritt zum Bestimmen einer Konzentration aus der elektrischen Leitfähigkeit ein optionaler Schritt zum Bestimmen der Konzentration. Es ist ausreichend, den Leitfähigkeitswert selbst zu bewerten.

Das erste bis dritte Temperaturmessmittel 21, 24, 28 sind nahe der ersten bis dritten Detektionseinheit 20a, 23a bzw. 27a angeordnet und das erste bis dritte Konzentrationsmessmittel 20, 23, 27 können die umgewandelten Konzentrationswerte durch die gemessenen Flüssigkeitstemperaturen korrigieren.

Wenn das Dialyseflüssigkeitszubereitungsgerät 1 im Dialyseflüssigkeitszubereitungsmodus ist, kann das erste Konzentrationsmessmittel 20 die elektrische Leitfähigkeit der Zubereitungsflüssigkeit der B-Flüssigkeit messen, die möglicherweise im ersten Puffertank 19, falls vorhanden, zubereitet wurde, und das zweite und dritte Konzentrationsmessmittel 23, 27 können die elektrische Leitfähigkeit der Dialyseflüssigkeit messen, die möglicherweise im zweiten Puffertank 22, falls vorhanden, zubereitet wurde.

Das Steuermittel 6 kann diese elektrischen Leitfähigkeiten der Zubereitungsflüssigkeit der B-Flüssigkeit und der Dialyseflüssigkeit, die durch das erste bis dritte Konzentrationsmessmittel 20, 23, 27 gemessen wurden, mit den zuvor registrierten elektrischen Leitfähigkeiten der Zubereitungsflüssigkeit der B-Flüssigkeit und der Dialyseflüssigkeit vergleichen und beurteilen, ob diese elektrischen Leitfähigkeiten abnormal sind.

Das Dialyseflüssigkeitszubereitungsgerät 1 kann ferner konfiguriert sein, im Diagnosemodus zum Durchführen einer Selbstprüfung zu laufen. Dieser Diagnosemodus kann zeitlich vor dem Zubereiten der Dialyseflüssigkeit liegen. Der Diagnosemodus kann Teil einer anfänglichen Testphase sein, in der verschiedene Funktionalitäten des Geräts, insbesondere des persönlichen Dialysegeräts 101.

Dabei kann das Steuermittel 6 eine Diagnoseeinheit 6a zum Durchführen der Selbstprüfung enthalten und in dieser Diagnoseeinheit 6a die Genauigkeit mindestens eines des ersten bis dritten Konzentrationsmessmittels 20, 23, 27 diagnostizieren.

Es wird angemerkt zu der vorliegenden Erfindungim Allgmeinen, dass, wenn der Audruck "Genauigkeit" ("accuracy" or "precision") verwendet wird dies auch einen Funktionalitätstest umfasst. Es ist nicht erforderlich, dass die Genauigkeit quantifiziert wird.

Figur 2, die eine zweite Ausführungsform zeigt, veranschaulicht eine Konfigurationszeichnung des sogenannten persönlichen Dialysegeräts 101. Wie oben bereits angegeben, hat dieses persönliche Dialysegerät 101 auch eine Funktion als Diagnoseflüssigkeitszubereitungsgerät 1, wie in Figur 1 dargestellt und im Zusammenhang mit Fugur 1 beschrieben, und kann die Diagnoseflüssigkeit zubereiten.

In der folgenden Ausführungsform werden in Bezug auf gemeinsame Bestandteile mit der obenstehenden ersten Ausführungsform Bezugszeichen verwendet, die sich aus einer Addition von 100 zu den Bezugszeichen ergeben, die in der ersten Ausführungsform verwendet wurden, und deren ausführliche Beschreibung wird unterlassen.

Das persönliche Dialysegerät 101 kann aufweisen oder dazu knfiguriert sein, aufzuweisen, beispielsweise durch Bereitstellen entsprechender Anschlussstücke für die Dialyseflüssigkeit, einen Dialysator F zum Durchführen der Dialyse zwischen Blut und Dialyseflüssigkeit, einen Blutkreislauf 107, der zwischen dem Dialysator F und einem Patienten angeschlossen wird, und die erste und zweite Kammer C1, C2 zum Zuleiten frischer Dialyseflüssigkeit zum Dialysator F und Sammeln verbrauchter Dialyseflüssigkeit enthalten oder konfiguriert sein, diese zu enthalten. Die erste und zweite Kammer C1, C2 können jeweils durch Membrane C1a, C2a mit Flexibilität in Beschickungskammern in welchen die frische Dialyseflüssigkeit gepoolt ist, und Sammelkammern, in welchen die verbrauchte Dialyseflüssigkeit gepoolt ist, geteilt sein. Die Beschickungskammern können an einen Wasserzuleitungskanal 102 und einen Flüssigkeitsbeschickungskanal 108 angeschlossen sein, der dem Dialysator F die frische Dialyseflüssigkeit zuleitet, und die Sammelkammern können an einen Abfallflüssigkeitskanal 105 und einen Sammelkanal 109 angeschlossen sein, der die verbrauchte Diagnoseflüssigkeit vom Dialysator F sammelt. Auf diesen Kanälen können jeweils verschiedene Ein/Aus-Solenoidventile bereitgestellt sein.

Der Wasserzuleitungskanal 2 in der ersten Ausführungsform entspricht dem Wasserzuleitungskanal 102 und Flüssigkeitsbeschickungskanal 108 in der zweiten Ausführungsform und die Detektionseinheit 127a des dritten Konzentrationsmessmittels 127, das dritte Temperaturmessmittel 128 und das erste Filter 125 können jeweils am Flüssigkeitsbeschickungskanal 108 bereitgestellt sein. Das dritte Konzentrationsmessmittel 127 und das dritte Temperaturmessmittel 128 können stromaufwärts des ersten Filters 125 am Flüssigkeitsbeschickungskanal 108 bereitgestellt sein und das zweite Filter 26 in der ersten Ausführungsform kann fehlen. Ferner kann die Flüssigkeitsbeschickungspumpe 131 am Sammelkanal 109 bereitgestellt sein.

Ein Umleitungskanal 110 kann zwischen dem ersten Filter 125 und dem Sammelkanal 109 bereitgestellt sein und durch Öffnen eines zweiten Ein/Aus-Ventils 132, das am Umleitungskanal 110 bereitgestellt ist, kann die Dialyseflüssigkeit, bei welcher eine schlechte Konzentration festgestellt wird, abgeleitet werden.

Das persönliche Dialysegerät 101 mit der oben beschriebenen Anordnung ist an sich weitgehend bekannt und daher wird auf eine ausführliche Beschreibung seines Verhaltens verzichtet. Ähnlich wie in der obenstehenden ersten Ausführungsform kann das persönliche Dialysegerät 101 die Genauigkeit des ersten bis dritten Konzentrationsmessmittels 120, 123, 127 mit einer Diagnoseeinheit 106a diagnostizieren, die in einem Steuermittel 106 bereitgestellt ist, und kann auch die Flüssigkeitsbeschickungsrate einer B-Flüssigkeitspumpe 118 und einer A-Flüssigkeitspumpe 116 diagnostizieren. In einer weiteren Ausführungsform kann der B-Flüssigkeitskanal stromabwärts des A-Flüssigkeitskanal anegschlossen sein, und es ist möglich, die Genaugkiet des ersten bis dritten Konzentrationsmessmittels 120, 123, 127 in diesem im Vergleich zur in Figur 2 gezeigten Anordnung umgekehrten Anschluss zu diagnostizieren.

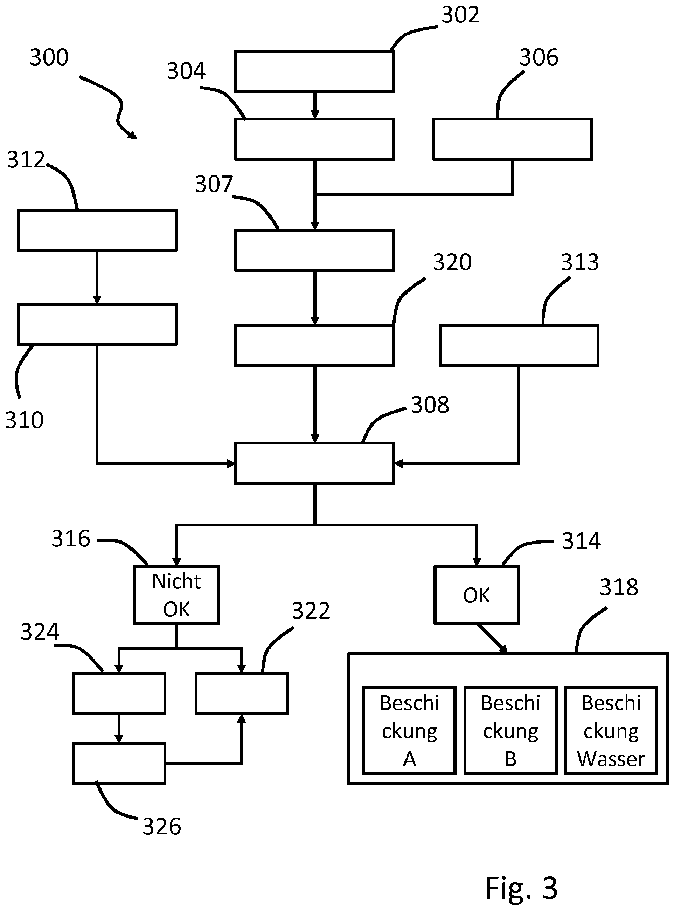

In der Folge wird das Verhalten des Dialyseflüssigkeitszubereitungsgeräts 1 mit der obenstehenden Konfiguration beschrieben. Ein Diagnoseverfahren 300 zum Diagnostizieren der Präzision mindestens eines des ersten bis dritten Konzentrationsmessmittels 20, 23, 27 wird beschrieben. Dieses ist schematisch in Figur 3 dargestellt. Unter Bezugnahme auf Figur 3 sind auch andere Schritte des Verfahrens beschrieben, aber aus der Beschreibung geht hervor, dass einige der Schritte optional sind. Wie oben angegeben, gilt die Beschreibung, die auf den Merkmalen der Ausführungsformen beruht, die unter Bezugnahme auf das Dialyseflüssigkeitszubereitungsgerät 1 beschrieben wurden, auch für Ausführungsformen des persönlichen Dialysegeräts 101.

Zu Beginn sind der A-Flüssigkeitskanal 3 und der B-Flüssigkeitskanal 4 an den A-Flüssigkeitstank 15 und den B-Flüssigkeitstank 17 angeschlossen, in welchem die A-Flüssigkeit bzw. B-Flüssigkeit, die zum Zubereiten der Dialyseflüssigkeit verwendet werden, bereitgestellt sind, und der Wasserzuleitungskanal 2 ist an eine Wasserquelle angeschlossen. In diesem Zustand kann ein Bediener ein Benutzerinteraktionsmittel, das in der Figur nicht dargestellt ist, bedienen, 302, um den Diagnosemodus zum Betätigen der Selbstprüfungsfunktion einzuleiten, und die Diagnoseeinheit 6a des Steuermittels 6 diagnostiziert die Genauigkeit des Konzentrationsmessmittels. Wie oben beschrieben, können weitere Prüfungen, beispielsweise der Ein/Aus-Solenoidventile, Pumpen und Messmittel, damit eingeleitet werden.

In Schritt 304 wird die A-Flüssigkeitspumpe 16 am A-Flüssigkeitskanal 3 betätigt. Zu diesem Zeitpunkt wird die B-Flüssigkeitspumpe 18 gestoppt. Optional kann parallel Wasser durch Betätigen der Wasserbeschickungspumpe 13 zugeleitet werden, 306, um eine verdünnte Lösung der A-Flüssigkeit zu erzeugen.

Dadurch fließt die A-Flüssigkeit, die aus dem A-Flüssigkeitstank 15 zugeleitet wird, durch den A-Flüssigkeitskanal 3 in den Wasserzuleitungskanal 2 und das Wasser, das zuvor in den Wasserzuleitungskanal 2 gefüllt wurde, wird zum Abfallflüssigkeitskanal 5 geschoben. Schließlich gelangt der Wasserzuleitungskanal 2 in einen Zustand, in dem die stromabwärts liegende Seite der Anschlussposition des A-Flüssigkeitskanal 3 nur mit der A-Flüssigkeit oder verdünnten A-Flüssigkeit gefüllt ist.

Wenn nach Betätigung der A-Flüssigkeitspumpe 16 ausreichend Zeit verstrichen ist, dass die A-Flüssigkeit zu mindestens einer der Detektionseinheiten 20a, 23a, 27a des zweiten und dritten Konzentrationsmessmittels 20, 23, 27 gelangt, misst das Steuermittel 6, Schritt 307, die elektrische Leitfähigkeit mit mindestens einer der zweiten bis dritten Detektionseinheiten 20a, 23a, 27a der zweiten bis dritten Konzentrationsmessmittel 20, 23, 27.

Dann vergleicht die Diagnoseeinheit 6a in Schritt 308 die gemessene elektrische Leitfähigkeit mit einem zuvor eingestellten Wert. Der zuvor eingestellte Wert kann ein Wert sein, der in einem Speicher 310 gespeichert ist. Der zuvor eingestellte Wert kann anhand einer Eingabe 312 eines Bedieners identifiziert werden, die die Art von A-Flüssigkeit identifiziert, wobei die Art von A-Flüssigkeit eine Identifizierung ihrer Inhaltsstoffe, eine automatische Identifizierung der Art von A-Flüssigkeit oder eines anderen Mittels ermöglicht. Dies kann auf der Basis einer Datenbank erfolgen, die im Speicher 310 bereitgestellt ist. Andere Parameter zum Bestimmen des zuvor eingestellten Werts können das Dosierungsverhältnis zwischen der A-Flüssigkeit und dem Wasser sein, wenn eine verdünnte A-Flüssigkeit gemessen werden soll. Die entsprechende Information kann einem Prozessor, beispielsweise der Diagnoseeinheit 6a oder einem anderen Prozessor, in dem Gerät bereitgestellt werden. Alternativ oder zusätzlich kann der zuvor eingestellte Wert ein Wert sein, der mit einer der Detektionseinheiten 20a, 23a, 27a des zweiten und dritten Konzentrationsmessmittels 20, 23, 27 gemessen wird, 313, das sich von dem zu diagnostizierenden Konzentrationsmessmittel 20, 23, 27 unterscheidet.

Wenn der Messwert der A-Flüssigkeit, unverdünnt oder verdünnt, der zu diesem Zeitpunkt gemessen wird, in einem vorgegebenen Fehlerbereich für den zuvor eingestellten Wert liegt, erfolgt eine Diagnose 314 in der Diagnoseeinheit 6a, dass er nicht abnormal ist, und wenn er den Fehlerbereich verlässt, erfolgt eine Diagnose 316. dass er abnormal ist.

Wenn das diagnostizierte der Konzentrationsmessmittel 20, 23, 27 abnormal ist, kann das Steuermittel 6 eine Warnung 322 für das richtige Konzentrationsmessmittel ausgeben. Die Warnung 322 kann eine Information sein, den Wartungsdienst zu informieren. Wenn das mindestens eine oder alle diagnostizierte Konzentrationsmessmittel 20, 23, 27 nicht abnormal sind, kann das Steuermittel 6 mit einem Betrieb, wie in der Folge beschrieben, fortfahren.

Das Diagnoseverfahren kann ferner, wenn eine Abweichung zwischen der gemessenen elektrischen Leitfähigkeit und dem zuvor eingestellten Wert einen vorgegebenen Schwellenwert übersteigt, ein Anzeigen 324 einer Nachricht, dass der Benutzer prüfen soll, ob die Art von A-Flüssigkeit, die als Eingabeparameter zum Bestimmen des zuvor eingestellten Werts verwendet wird, einer Art von A-Flüssigkeit der A-Flüssigkeit entspricht, auf der Anzeige umfassen und bei Betätigung eines Benutzerinteraktionsmittels zur Bestätigung 326, dass die Art von A-Flüssigkeit, die als Eingabeparameter zum Bestimmen des zuvor eingestellten Werts verwendet wird, der Art von A-Flüssigkeit der A-Flüssigkeit entspricht, ein Anzeigen 322 einer Fehlernachricht, die eine Fehlfunktion der Dialyseflüssigkeitszubereitung angibt, auf der Anzeige der Dialyseflüssigkeitszubereitung.

Wenn festgestellt wurde 314, dass kein abnormaler Zustand für das mindestens eine diagnostizierte Konzentrationsmessmittel 20, 23, 27 vorliegt, kann das Dialyseflüssigkeitszubereitungsgerät 1 fortfahren und in einen Dialyseflüssigkeitszubereitungsmodus oder einen Behandlungsmodus 318 eintreten, der ein Mischen von A-Flüssigkeit, B-Flüssigkeit und Wasser zum Zubereiten der Dialyseflüssigkeit enthält.

Zusätzlich oder alternativ kann das Steuermittel 6 kontinuierlich die elektrische Leitfähigkeit mit mindestens einer der Detektionseinheiten 20a, 23a, 27a messen. Nachdem die A-Flüssigkeitspumpe 16 begonnen hat, die A-Flüssigkeit zuzuleiten, und das Steuermittel 6 kann den Zeitpunkt messen, bevor die elektrische Leitfähigkeit den obengenannten vorgegebenen Wert erreicht, das heißt, den Zeitpunkt, bevor die A-Flüssigkeit die entsprechende Detektionseinheit 20a, 23a, 27a erreicht. Dann kann die Diagnoseeinheit 6a den gemessenen Zielzeitpunkt der A-Flüssigkeit mit einem zuvor eingestellten Standardzeitpunkt vergleichen und beurteilen, ob die A-Flüssigkeitspumpe 16 abnormal 320 ist. Wenn der Zielzeitpunkt später als der Standardzeitpunkt ist, ist die Flüssigkeitsbeschickungsrate der A-Flüssigkeitspumpe 16 unzureichend, und wenn er früher ist, ist die Flüssigkeitsbeschickungsrate höher als die Spezifikation.

Falls das Messen der elektrischen Leitfähigkeit ein Messen nur der A-Flüssigkeit mit den entsprechenden Detektionseinheiten 20a, 23a, 27a des ersten bis dritten Konzentrationsmessmittels 20, 23, 27 war, kann das Steuermittel 6 das erste Ein/Aus-Ventil 11 am Wasserzuleitungskanal 2 öffnen und kann ferner die Wasserpumpe 13 betätigen, so dass das Wasser bei einer vorgegebenen Durchflussrate in den Wasserzuleitungskanal 2 fließt. Bei dieser Gelegenheit kann die A-Flüssigkeitspumpe 16 die Flüssigkeitsbeschickung bei einer spezifizierten Durchflussrate durchführen und dadurch werden die A-Flüssigkeit und das Wasser im zweiten Puffertank 22, falls vorhanden, am Wasserzuleitungskanal 2 gemischt, so dass die Zubereitungsflüssigkeit der A-Flüssigkeit bei einer vorgegebenen Konzentration zubereitet wird. Diese Zubereitungsflüssigkeit der A-Flüssigkeit kann zum Abfallflüssigkeitskanal 5 fließen.

Ähnlich wie im Fall eines Zuleitens nur der A-Flüssigkeit misst das Steuermittel 6 die elektrische Leitfähigkeit mit mindestens einer der ersten bis dritten Detektionseinheit 20a, 23a, 27a und die Diagnoseeinheit 6a vergleicht den Messwert mit einem zuvor eingestellten Wert, der der Konzentration der Zubereitungsflüssigkeit der A-Flüssigkeit entspricht.

Durch derartiges Messen der Konzentrationen der A-Flüssigkeit und der Zubereitungsflüssigkeit der A-Flüssigkeit, das heißt, der Flüssigkeiten mit unterschiedlichen elektrischen Leitfähigkeiten, ist es möglich, das erste bis dritte Konzentrationsmessmittel 20, 23, 27 exakter zu diagnostizieren.

Diese zweite Messung an der verdünnten A-Flüssigkeit entspricht dem oben beschriebenen Prozess zum Diagnostizieren mittels der verdünnten A-Flüssigkeit und kann daher alleine oder in Kombination mit dem Diagnoseverfahren, das die A-Flüssigkeit allein verwendet, durchgeführt werden.

Nach dem derartigen Messen der elektrischen Leitfähigkeit der Zubereitungsflüssigkeit der A-Flüssigkeit mit den Detektionseinheiten 20a, 23a, 27a des ersten bis dritten Konzentrationsmessmittels 20, 23, 27 kann das Steuermittel 6 die A-Flüssigkeitspumpe 16 am A-Flüssigkeitskanal 3 zum Zuleiten der A-Flüssigkeit in den Wasserzuleitungskanal 2 betätigen und kann ferner die B-Flüssigkeitspumpe 18 betätigen und dadurch wird im zweiten Puffertank 22, falls vorhanden, der stromabwärts der Anschlussposition des A-Flüssigkeitskanals 3 positioniert ist, die B-Flüssigkeit der Zubereitungsflüssigkeit der A-Flüssigkeit beigemischt, so dass die Dialyseflüssigkeit zubereitet wird. Die Dialyseflüssigkeit kann im Wasserzuleitungskanal 2 zum Abfallflüssigkeitskanal 5 fließen. Das Steuermittel 6 kann die elektrische Leitfähigkeit der Dialyseflüssigkeit mit der zweiten Detektionseinheit 23a des zweiten Konzentrationsmessmittels 23 und der dritten Detektionseinheit 27a des dritten Konzentrationsmessmittels 27 messen, diese mit einem zuvor eingestellten Wert als die elektrische Leitfähigkeit der Dialyseflüssigkeit vergleichen und beurteilen, ob die Konzentration der Dialyseflüssigkeit gut oder schlecht ist.

Ferner ist durch Messen der Zeit, nach der die B-Flüssigkeitspumpe 18 mit dem Zuleiten der B-Flüssigkeit beginnen kann, und bevor die zweite Detektionseinheit 23a die elektrische Leitfähigkeit entsprechend der vorgegebenen Konzentration der Dialyseflüssigkeit messen kann, möglich, die Flüssigkeitsbeschickungsrate der B-Flüssigkeitspumpe 16 zu diagnostizieren.

Somit ist die Diagnose der Genauigkeit des ersten bis dritten Konzentrationsmessmittels 20, 23, 27 beendet und wenn die anderen Geräte auch nicht als abnormal beurteilt werden, beendet das Steuermittel 6 die Diagnose des Dialyseflüssigkeitszubereitungsgeräts 1. Das Dialyseflüssigkeitszubereitungsgerät 1 wechselt vom Diagnosemodus in den Zubereitungsmodus und bereitet anschließend die Dialyseflüssigkeit zu, 318. Dieser Wechsel kann auch durch einen Anwender initieert werden.

Somit ist es im Dialyseflüssigkeitszubereitungsgerät 1 gemäß der obengenannten Ausführungsform durch die Diagnoseeinheit 6a, die im Steuermittel 6 bereitgestellt ist, möglich, die Genauigkeit wenigstens eines des ersten bis dritten Konzentrationsmessmittels 20, 23, 27 automatisch zu diagnostizieren.

Obwohl in der obenstehenden Ausführungsform der B-Flüssigkeitskanal 4 stromaufwärts des A-Flüssigkeitskanals 3 am Wasserzuleitungskanal 2 angeschlossen ist, kann er stromabwärts des A-Flüssigkeitskanal 3 angeschlossen sein. Das heißt, es ist eine Konfiguration möglich, dass in Figur 1 das Bezugszeichen 3 den B-Flüssigkeitskanal bezeichnet, das Bezugszeichen 4 den A-Flüssigkeitskanal bezeichnet, die Bezugszeichen 15, 16 den B-Flüssigkeitstank bzw. die B-Flüssigkeitspumpe bezeichnen und die Bezugszeichen 17, 18 den A-Flüssigkeitstank bzw. den A-Flüssigkeitspumpe bezeichnen.

Alle Ausführungsformen können mehr als ein Konzentrationsmessmittel mit der entsprechenden Detektionseinheit aufweisen. Es soll betont werden, dass das persönliche Dialysegerät genau ein Konzentrationsmessmittel mit der entsprechenden Detektionseinheit stromaufwärts des Dialysators haben kann. Für das persönliche Dialysegerät ist dies günstig, da dies die Kosten der Maschine weiter senkt.

## Patentansprüche

1. Diagnoseverfahren für ein Dialyseflüssigkeitszubereitungsgerät (1, 101), das umfasst:
einen Wasserzuleitungskanal (2, 102) zum Zuleiten von Wasser,
einen A-Flüssigkeitskanal (3, 103) zum Zuleiten einer A-Flüssigkeit, die Natriumchlorid als einen Hauptinhaltsstoff gemeinsam mit mindestens einem anderen Inhaltsstoff enthält, wobei der A-Flüssigkeitskanal (3, 103) an den Wasserzuleitungskanal (2, 102) angeschlossen ist,
einen B-Flüssigkeitskanal (4, 104) zum Zuleiten einer B-Flüssigkeit, die aus einer wässrigen Natriumbicarbonatlösung besteht, wobei der B-Flüssigkeitskanal (4, 104) an den Wasserzuleitungskanal (2, 102) angeschlossen ist, und Konzentrationsmessmittel (20, 23, 27, 120, 123, 127), die mit einer Detektionseinheit (20a, 23a, 27a, 120a, 123a, 127a) stromabwärts der Anschlusspositionen der A-Flüssigkeitskanal (3, 103) am Wasserzuleitungskanal (2, 102) bereitgestellt sind,
wobei das Diagnoseverfahren umfasst
Mischen des Wassers, der A-Flüssigkeit und der B-Flüssigkeit jeweils in vorgegebenen Anteilen, um eine Dialyseflüssigkeit zuzubereiten, und Messen einer Konzentration der zubereiteten Dialyseflüssigkeit mit dem Konzentrationsmessmittel (20, 23, 27, 120, 123, 127), **gekennzeichnet durch,** vor dem Zubereiten der Dialyseflüssigkeit, Zuleiten der A-Flüssigkeit aus dem A-Flüssigkeitskanal (3, 103) in den Wasserzuleitungskanal (2, 102), so dass nur die A-Flüssigkeit in den Wasserzuleitungskanal (2, 102) fließt, und Diagnostizieren einer Messgenauigkeit des Konzentrationsmessmittels (20, 23, 27, 120, 123, 127) durch Messen einer elektrischen Leitfähigkeit der A-Flüssigkeit, unverdünnt oder verdünnt, mit dem Konzentrationsmessmittel (20, 23, 27, 120, 123, 127) und dann Vergleichen der gemessenen elektrischen Leitfähigkeit der A-Flüssigkeit bzw. der verdünnten A-Flüssigkeit mit einem zuvor eingestellten Wert.

2. Diagnoseverfahren für das Dialyseflüssigkeitszubereitungsgerät (1, 101) nach Anspruch 1, des Weiteren umfassend:
beim Messen der elektrischen Leitfähigkeit der A-Flüssigkeit, Messen einer Zeit, nachdem eine A-Flüssigkeitspumpe (16, 116) mit einer Flüssigkeitsbeschickung der A-Flüssigkeit begonnen hat und bevor das Konzentrationsmessmittel (20, 23, 27, 120, 123, 127) eine Konzentration der A-Flüssigkeit misst, wobei die A-Flüssigkeitspumpe (16, 116) am A-Flüssigkeitskanal bereitgestellt ist (3, 103) und die A-Flüssigkeit in den Wasserzuleitungskanal (2, 102) leitet, und dadurch Diagnostizieren der Flüssigkeitsbeschickungsgenauigkeit der A-Flüssigkeitspumpe (16, 116).

3. Diagnoseverfahren für das Dialyseflüssigkeitszubereitungsgerät (1, 101) nach einem der Ansprüche 1 und 2, des Weiteren umfassend:
zur Herstellung der verdünnten A-Flüssigkeit (3, 103), Mischen des Wassers und der A-Flüssigkeit, um eine Zubereitungsflüssigkeit der A-Flüssigkeit zuzubereiten, indem das Wasser in den Wasserzuleitungskanal (2, 102) geleitet wird.

4. Diagnoseverfahren für das Dialyseflüssigkeitszubereitungsgerät (1, 101) nach einem der vorangehenden Ansprüche, des Weiteren umfassend:
wenn eine Abweichung zwischen der gemessenen elektrischen Leitfähigkeit und dem zuvor eingestellten Wert einen vorgegebenen Schwellenwert übersteigt, Anzeigen (324) einer Nachricht, dass der Benutzer prüfen soll, ob eine Art von A-Flüssigkeit, die als Eingabeparameter zum Bestimmen des zuvor eingestellten Werts verwendet wird, einer Art von A-Flüssigkeit der A-Flüssigkeit, die mit dem A-Flüssigkeitskanal (3, 103) verbunden ist, entspricht, auf einer Anzeige des Dialyseflüssigkeitszubereitungsgeräts.

5. Diagnoseverfahren für das Dialyseflüssigkeitszubereitungsgerät (1, 101) nach Anspruch 4, des Weiteren umfassend:
Anzeigen (322) einer Fehlernachricht auf der Anzeige des Dialyseflüssigkeitszubereitungsgeräts, die eine Fehlfunktion des Dialyseflüssigkeitszubereitungsgeräts angibt, wenn eine Art von A-Flüssigkeit, die als Eingabeparameter zum Bestimmen des zuvor eingestellten Werts verwendet wird, einer Art von A-Flüssigkeit der A-Flüssigkeit, die mit dem A-Flüssigkeitskanal (3, 103) verbunden ist, entspricht.

6. Diagnoseverfahren für das Dialyseflüssigkeitszubereitungsgerät (1, 101) nach einem der vorangehenden Ansprüche, des Weiteren umfassend:
Bereitstellen eines Benutzerinteraktionsmittels zum Bestätigen (326), dass die Art von A-Flüssigkeit, die als Eingabeparameter zum Bestimmen des zuvor eingestellten Werts verwendet wird, der Art von A-Flüssigkeit der A-Flüssigkeit, die mit dem A-Flüssigkeitskanal (3, 103) verbunden ist, entspricht, und Anzeigen (322) einer Warnnachricht auf der Anzeige des Dialyseflüssigkeitszubereitungsgeräts (1, 101), wenn das Benutzerinteraktionsmittel vom Benutzer aktiviert wurde.

7. Diagnoseverfahren für das Dialyseflüssigkeitszubereitungsgerät (1, 101) nach einem der vorangehenden Ansprüche, des Weiteren umfassend, dass der zuvor eingestellte Wert ein Wert ist, der auf einer weiteren elektrischen Leitfähigkeit beruht, die von einem weiteren Detektionsmittel eines weiteren Konzentrationsmessmittels (20, 23, 27, 120, 123, 127) gemessen wird, das im A-Flüssigkeitskanal (3, 103) oder im Wasserzuleitungskanal (2, 102) stromabwärts des Anschlusses des A-Flüssigkeitskanals (3, 103) am Wasserzuleitungskanal (2, 102) angeordnet ist.

8. Dialyseflüssigkeitszubereitungsgerät (1, 101)aufweisend:
einen Wasserzuleitungskanal (2, 102) zum Zuleiten von Wasser,
eine Wasserbeschickungspumpe, die am Wasserzuleitungskanal (2, 102) bereitgestellt ist,
einen A-Flüssigkeitskanal (3, 103) zum Zuleiten einer A-Flüssigkeit, die Natriumchlorid als einen Hauptinhaltsstoff gemeinsam mit mindestens einem anderen Inhaltsstoff enthält, wobei der A-Flüssigkeitskanal (3, 103) an den Wasserzuleitungskanal (2, 102) angeschlossen ist,
eine A-Flüssigkeitspumpe (16, 116), die am A-Flüssigkeitskanal bereitgestellt ist (3, 103),
einen B-Flüssigkeitskanal (4, 104) zum Zuleiten einer B-Flüssigkeit, die aus einer wässrigen Natriumbicarbonatlösung besteht, wobei der B-Flüssigkeitskanal (4, 104) an den Wasserzuleitungskanal (2, 102) angeschlossen ist,
eine B-Flüssigkeitspumpe (18, 118), die am B-Flüssigkeitskanal bereitgestellt ist (4, 104),
Konzentrationsmessmittel (20, 23, 27, 120, 123, 127), die mit einer Detektionseinheit (20a, 23a, 27a, 120a, 123a, 127a) stromabwärts der Anschlusspositionen des A-Flüssigkeitskanals (3, 103) am Wasserzuleitungskanal (2, 102) bereitgestellt sind, und
Steuermittel (6, 106) zum Steuern von Betätigungen der Pumpen,
wobei das Steuermittel (6, 106) des Dialyseflüssigkeitszubereitungsgeräts (1, 101) zum Steuern eines Mischens des Wassers, der A-Flüssigkeit und der B-Flüssigkeit in vorgegebenen Anteilen konfiguriert ist, um eine Dialyseflüssigkeit zuzubereiten, und wobei das Steuermittel (6, 106) mit einer Diagnoseeinheit (6a, 106a) bereitgestellt ist, **dadurch gekennzeichnet, dass**
die Diagnoseeinheit (6a, 106a) zum Diagnostizieren der Präzision des Konzentrationsmessmittels (20, 23, 27, 120, 123, 127) durch Betätigen der A-Flüssigkeitspumpe (16, 116) konfiguriert ist, um eine Flüssigkeitsbeschickung nur der A-Flüssigkeit in den Wasserzuleitungskanal (2, 102) durchzuführen, ohne eine Flüssigkeitsbeschickung mit der B-Flüssigkeitspumpe (18, 118) durchzuführen, wobei die Diagnoseeinheit (6a, 106a) zum Diagnostizieren der Genauigkeit des Konzentrationsmessmittels (20, 23, 27, 120, 123, 127) durch einen Vergleich einer elektrischen Leitfähigkeit der A-Flüssigkeit, unverdünnt oder verdünnt, die durch die Detektionseinheit (20a, 23a, 27a, 120a, 123a, 127a) des Konzentrationsmessmittels (20, 23, 27, 120, 123, 127) gemessen wird, mit einem zuvor eingestellten Wert konfiguriert ist.

9. Dialyseflüssigkeitszubereitungsgerät (1, 101) nach Anspruch 8, das des Weiteren konfiguriert ist zum,
beim Messen der elektrischen Leitfähigkeit der A-Flüssigkeit, Messen einer Zeit, nachdem die A-Flüssigkeitspumpe (16, 116) begonnen hat, die A-Flüssigkeit zuzuleiten, und bevor das Konzentrationsmessmittel (20, 23, 27, 120, 123, 127) die Konzentration der A-Flüssigkeit misst, und dadurch Diagnostizieren einer Flüssigkeitsbeschickungsgenauigkeit der B-Flüssigkeitspumpe (18, 118).

10. Dialyseflüssigkeitszubereitungsgerät (1, 101) nach einem der Ansprüche 8 und 9, das des Weiteren konfiguriert ist zum,
zur Herstellung der verdünnten A-Flüssigkeit (3, 103), Mischen des Wassers und der A-Flüssigkeit, um eine Zubereitungsflüssigkeit der A-Flüssigkeit zuzubereiten, indem das Wasser in den Wasserzuleitungskanal (2, 102) geleitet wird.

11. Dialyseflüssigkeitszubereitungsgerät (1, 101) nach einem der Ansprüche 8 bis 10,
des Weiteren umfassend eine Anzeige, und
konfiguriert zum Anzeigen (324), wenn eine Abweichung zwischen der gemessenen elektrischen Leitfähigkeit und dem zuvor eingestellten Wert einen vorgegebenen Schwellenwert übersteigt, einer Nachricht auf der Anzeige, dass der Benutzer prüfen soll, ob eine Art von A-Flüssigkeit, die als Eingabeparameter zum Bestimmen des zuvor eingestellten Werts verwendet wird, einer Art von A-Flüssigkeit der A-Flüssigkeit, die mit dem A-Flüssigkeitskanal (3, 103) verbunden ist, entspricht.

12. Dialyseflüssigkeitszubereitungsgerät (1, 101) nach Anspruch 11, des Weiteren konfiguriert zum Anzeigen einer Fehlernachricht auf der Anzeige (322), die eine Fehlfunktion des Dialyseflüssigkeitszubereitungsgeräts (1, 101) angibt, wenn eine Art von A-Flüssigkeit, die als Eingabeparameter zum Bestimmen des zuvor eingestellten Werts verwendet wird, einer Art von A-Flüssigkeit der A-Flüssigkeit, die mit dem A-Flüssigkeitskanal (3, 103) verbunden ist, entspricht.

13. Dialyseflüssigkeitszubereitungsgerät (1, 101) Anspruch 12, umfassend
ein Benutzerinteraktionsmittel und
des Weiteren konfiguriert, bei Betätigung des Benutzerinteraktionsmittels zum Bestätigen (326), dass die Art von A-Flüssigkeit, die als Eingabeparameter zum Bestimmen des zuvor eingestellten Werts verwendet wird, der Art von A-Flüssigkeit der A-Flüssigkeit, die mit dem A-Flüssigkeitskanal (3, 103) verbunden ist, entspricht, zum Anzeigen (322) einer Fehlernachricht auf der Anzeige der Dialyseflüssigkeitszubereitung, die eine Fehlfunktion der Dialyseflüssigkeitszubereitunggeräts angibt.

14. Dialyseflüssigkeitszubereitungsgerät (1, 101) nach einem der Ansprüche 8 bis 13, des Weiteren umfassend
eine weitere Detektionseinheit eines weiteren Konzentrationsmessmittels (20, 23, 27, 120, 123, 127), das im A-Flüssigkeitskanal (3, 103) oder im Wasserzuleitungskanal (2, 102) stromabwärts des Anschlusses des A-Flüssigkeitskanals (3, 103) am Wasserzuleitungskanal (2, 102) angeordnet ist, wobei das Dialyseflüssigkeitszubereitungsgerät (1, 101) konfiguriert ist, als zuvor eingestellten Wert einen Wert auf der Basis der weiteren elektrischen Leitfähigkeit zu verwenden, die durch das weitere Detektionsmittel gemessen wird.

## Claims

1. Diagnostic method for a dialysis fluid preparation device (1, 101), the latter comprising:
a water supply channel (2, 102) for supplying water, an A-fluid channel (3, 103) for supplying an A-fluid containing sodium chloride as a main ingredient, together with at least one other ingredient, the A-fluid channel (3, 103) being connected to the water supply channel (2, 102),
a B-fluid channel (4, 104) for supplying a B-fluid consisting of an aqueous sodium bicarbonate solution, with the B-fluid channel (4, 104) being connected to the water supply channel (2, 102), and
concentration measuring means (20, 23, 27, 120, 123, 127), provided with a detection unit (20a, 23a, 27a, 120a, 123a, 127a) downstream of the connection positions of the A-fluid channel (3, 103) on the water supply channel (2, 102),
the diagnostic method comprising:
mixing the water, the A-fluid and the B-fluid, each in predetermined proportions, in order to prepare a dialysis fluid, and
measuring a concentration of the prepared dialysis fluid using the concentration measuring means (20, 23, 27, 120, 123, 127), **characterized by,**
prior to the preparation of the dialysis fluid, supplying the A-fluid from the A-fluid channel (3, 103) to the water supply channel (2, 102) such that it is only A-fluid flowing in the water supply channel (2, 102), and diagnosing a measurement accuracy of the concentration measuring means (20, 23, 27, 120, 123, 127) by measuring an electrical conductivity of the A-fluid, be it undiluted or diluted, using the concentration measuring means (20, 23, 27, 120, 123, 127) and subsequently comparing the measured electrical conductivity of the A-fluid or the undiluted A-fluid with a value set in advance.

2. Diagnostic method for the dialysis fluid preparation device (1, 101) according to Claim 1, further comprising: within the scope of measuring the electrical conductivity of the A-fluid, measuring a time after an A-fluid pump (16, 116) has started a fluid feed of the A-fluid and before the concentration measuring means (20, 23, 27, 120, 123, 127) measures a concentration of the A-fluid, with the A-fluid pump (16, 116) being provided on the A-fluid channel (3, 103) and guiding the A-fluid into the water supply channel (2, 102), and thereby diagnosing the fluid feed accuracy of the A-fluid pump (16, 116).

3. Diagnostic method for the dialysis fluid preparation device (1, 101) according to either of Claims 1 and 2, further comprising:
for the purpose of producing the diluted A-fluid (3, 103), mixing the water and the A-fluid in order to prepare a preparation fluid of the A-fluid by virtue of the water being guided into the water supply channel (2, 102).

4. Diagnostic method for the dialysis fluid preparation device (1, 101) according to any of the preceding claims, further comprising:
should a deviation between the measured electrical conductivity and the value set in advance exceed a predetermined threshold value, displaying (324) a message to the effect that the user should check whether a type of A-fluid used as an input parameter for determining the value set in advance corresponds to a type of A-fluid of the A-fluid connected to the A-fluid channel (3, 103), on a display of the dialysis fluid preparation device.

5. Diagnostic method for the dialysis fluid preparation device (1, 101) according to Claim 4, further comprising: displaying (322) an error message indicating a malfunction of the dialysis fluid preparation device on the display of the dialysis fluid preparation device if a type of A-fluid used as an input parameter for determining the value set in advance corresponds to a type of A-fluid of the A-fluid connected to the A-fluid channel (3, 103).

6. Diagnostic method for the dialysis fluid preparation device (1, 101) according to any of the preceding claims, further comprising:
providing a user interaction means for confirming (326) that the type of A-fluid used as an input parameter for determining the value set in advance corresponds to the type of A-fluid of the A-fluid connected to the A-fluid channel (3, 103), and displaying (322) a warning message on the display of the dialysis fluid preparation device (1, 101) if the user interaction means was activated by the user.

7. Diagnostic method for the dialysis fluid preparation device (1, 101) according to any of the preceding claims, further comprising the fact that the value set in advance is a value based on a further electrical conductivity measured by a further detection means of a further concentration measuring means (20, 23, 27, 120, 123, 127) arranged in the A-fluid channel (3, 103) or in the water supply channel (2, 102) downstream of the connection of the A-fluid channel (3, 103) on the water supply channel (2, 102).

8. Dialysis fluid preparation device (1, 101), having:
a water supply channel (2, 102) for supplying water,
a water feed pump provided on the water supply channel (2, 102),
an A-fluid channel (3, 103) for supplying an A-fluid containing sodium chloride as a main ingredient, together with at least one other ingredient, the A-fluid channel (3, 103) being connected to the water supply channel (2, 102),
an A-fluid pump (16, 116) provided on the A-fluid channel (3, 103),
a B-fluid channel (4, 104) for supplying a B-fluid consisting of an aqueous sodium bicarbonate solution, with the B-fluid channel (4, 104) being connected to the water supply channel (2, 102),
a B-fluid pump (18, 118) provided on the B-fluid channel (4, 104),
concentration measuring means (20, 23, 27, 120, 123, 127), provided with a detection unit (20a, 23a, 27a, 120a, 123a, 127a) downstream of the connection positions of the A-fluid channel (3, 103) on the water supply channel (2, 102), and
control means (6, 106) for controlling actuations of the pumps,
with the control means (6, 106) of the dialysis fluid preparation device (1, 101) being configured to control mixing of the water, the A-fluid and the B-fluid in predetermined proportions in order to prepare a dialysis fluid, and with the control means (6, 106) being provided with a diagnostic unit (6a, 106a), **characterized in that** the diagnostic unit (6a, 106a) is configured to diagnose the precision of the concentration measuring means (20, 23, 27, 120, 123, 127) by actuating the A-fluid pump (16, 116) in order to perform a fluid feed of only the A-fluid into the water supply channel (2, 102) without performing a fluid feed with the B-fluid pump (18, 118), with the diagnostic unit (6a, 106a) being configured to diagnose the accuracy of the concentration measuring means (20, 23, 27, 120, 123, 127) by comparing an electrical conductivity of the A-fluid, be it undiluted or diluted and as measured by the detection unit (20a, 23a, 27a, 120a, 123a, 127a) of the concentration measuring means (20, 23, 27, 120, 123, 127), with a value set in advance.

9. Dialysis fluid preparation device (1, 101) according to Claim 8, further configured,
within the scope of measuring the electrical conductivity of the A-fluid, to measure a time after the A-fluid pump (16, 116) has started to supply the A-fluid and before the concentration measuring means (20, 23, 27, 120, 123, 127) measures the concentration of the A-fluid, and to thereby diagnose a fluid feed accuracy of the B-fluid pump (18, 118).

10. Dialysis fluid preparation device (1, 101) according to either of Claims 8 and 9, further configured,
for the purpose of producing the diluted A-fluid (3, 103), to mix the water and the A-fluid in order to prepare a preparation fluid of the A-fluid by virtue of the water being guided into the water supply channel (2, 102).

11. Dialysis fluid preparation device (1, 101) according to any of Claims 8 to 10,
further comprising a display and,
should a deviation between the measured electrical conductivity and the value set in advance exceed a predetermined threshold value, being configured to display (324) a message on the display to the effect that the user should check whether a type of A-fluid used as an input parameter for determining the value set in advance corresponds to a type of A-fluid of the A-fluid connected to the A-fluid channel (3, 103).

12. Dialysis fluid preparation device (1, 101) according to Claim 11, further configured to display an error message on the display (322), said error message indicating a malfunction of the dialysis fluid preparation device (1, 101) if a type of A-fluid used as an input parameter for determining the value set in advance corresponds to a type of A-fluid of the A-fluid connected to the A-fluid channel (3, 103).

13. Dialysis fluid preparation device (1, 101) according to Claim 12, comprising
a user interaction means and further being configured, upon actuation of the user interaction means for the purpose of confirming (326) that the type of A-fluid used as an input parameter for determining the value set in advance corresponds to the type of A-fluid of the A-fluid connected to the A-fluid channel (3, 103), to display (322) an error message on the display of the dialysis fluid preparation device indicating a malfunction of the dialysis fluid preparation device.

14. Dialysis fluid preparation device (1, 101) according to any of Claims 8 to 13, further comprising
a further detection unit of a further concentration measuring means (20, 23, 27, 120, 123, 127) arranged in the A-fluid channel (3, 103) or in the water supply channel (2, 102) downstream of the connection of the A-fluid channel (3, 103) on the water supply channel (2, 102),
with the dialysis fluid preparation device (1, 101) being configured to use a value based on the further electrical conductivity measured by the further detection means as the value set in advance.

## Revendications

1. Procédé de diagnostic destiné à un dispositif de préparation de liquide de dialyse (1,101) qui comprend :
un conduit d'alimentation en eau (2, 102) destiné à fournir de l'eau, un conduit de liquide A (3, 103) destiné à fournir un liquide A qui contient du chlorure de sodium comme ingrédient principal conjointement avec au moins un autre ingrédient, le conduit de liquide A (3, 103) étant raccordé au conduit d'alimentation en eau (2, 102),
un conduit de liquide B (4, 104) destiné à fournir un liquide B qui comprend une solution aqueuse de bicarbonate de sodium, le conduit de liquide B (4, 104) étant raccordé au conduit d'alimentation en eau (2, 102), et
des moyens de mesure de concentration (20, 23, 27, 120, 123, 127) qui sont équipés d'une unité de détection (20a, 23a, 27a, 120a, 123a, 127a) sur le conduit d'alimentation en eau (2, 102) en aval des positions de raccordement du conduit de liquide A (3, 103),
le procédé de diagnostic comprenant les étapes suivantes
mélanger l'eau, le liquide A et le liquide B à chaque fois dans des proportions spécifiées afin de préparer un liquide de dialyse, et
mesurer une concentration du liquide de dialyse préparé avec le moyen de mesure de concentration (20, 23, 27, 120, 123, 127), **caractérisé par** les étapes suivantes :
avant de préparer le liquide de dialyse, amener le liquide A depuis le conduit de liquide A (3, 103) jusque dans le conduit d'alimentation en eau (2, 102) de sorte que seul le liquide A s'écoule dans le conduit d'alimentation en eau (2, 102), et
diagnostiquer une précision de mesure du moyen de mesure de concentration (20, 23, 27, 120, 123, 127) par mesure d'une conductivité électrique du liquide A, dilué ou non dilué, avec le moyen de mesure de concentration (20, 23, 27, 120, 123, 127), puis comparer la conductivité électrique mesurée du liquide A, respectivement du liquide A dilué, à une valeur préalablement réglée.

2. Procédé de diagnostic du dispositif de préparation de liquide de dialyse (1, 101) selon la revendication 1, ledit procédé comprenant en outre les étapes suivantes :
lors de la mesure de la conductivité électrique du liquide A, mesurer un temps après qu'une pompe de liquide A (16, 116) a commencé l'alimentation en liquide A et avant que le moyen de mesure de concentration (20, 23, 27, 120, 123, 127) ne mesure une concentration en liquide A, la pompe de liquide A (16, 116) étant fournie au niveau du conduit de liquide A (3, 103) et
diriger le liquide A dans le conduit d'alimentation en eau (2, 102), et
diagnostiquer ainsi la précision d'alimentation en liquide de la pompe de liquide A (16, 116).

3. Procédé de diagnostic destiné au dispositif de préparation de liquide de dialyse (1, 101) selon l'une des revendications 1 et 2, ledit procédé comprenant en outre l'étape suivante :
pour préparer le liquide A dilué (3, 103), mélanger l'eau et le liquide A afin de préparer un liquide de préparation du liquide A par introduction d'eau dans le conduit d'alimentation en eau (2, 102).

4. Procédé de diagnostic destiné au dispositif de préparation de liquide de dialyse (1, 101) selon l'une des revendications précédentes, ledit procédé comprenant en outre l'étape suivante :
si un écart entre la conductivité électrique mesurée et la valeur préalablement réglée dépasse une valeur seuil spécifiée, afficher (324) sur un afficheur du dispositif de préparation de liquide de dialyse un message indiquant que l'utilisateur doit vérifier si un type de liquide A, qui est utilisé comme paramètre d'entrée pour déterminer la valeur préalablement réglée, correspond à un type de liquide A du liquide A qui est relié au conduit de liquide A (3, 103).

5. Procédé de diagnostic destiné au dispositif de préparation de liquide de dialyse (1, 101) selon la revendication 4, ledit procédé comprenant en outre l'étape suivante :
afficher (322) sur l'affichage du dispositif de préparation de liquide de dialyse un message d'erreur indiquant un dysfonctionnement du dispositif de préparation de liquide de dialyse lorsqu'un type de liquide A, qui est utilisé comme paramètre d'entrée pour déterminer la valeur préalablement réglée, correspond à un type de liquide A du liquide A qui est relié au conduit de liquide A (3, 103).

6. Procédé de diagnostic destiné au dispositif de préparation de liquide de dialyse (1, 101) selon l'une des revendications précédentes, ledit procédé comprenant en outre les étapes suivantes :
fournir un moyen d'interaction utilisateur pour confirmer (326) que le type de liquide A, qui est utilisé comme paramètre d'entrée pour déterminer la valeur préalablement réglée, correspond au type de liquide A du liquide A qui est relié au conduit de liquide A (3, 103), et
afficher (322) un message d'avertissement sur l'afficheur du dispositif de préparation de liquide de dialyse (1, 101) lorsque le moyen d'interaction utilisateur a été activé par l'utilisateur.

7. Procédé de diagnostic destiné au dispositif de préparation de liquide de dialyse (1, 101) selon l'une des revendications précédentes, ledit procédé comprenant en outre l'étape dans laquelle la valeur préalablement réglée est une valeur qui est basée sur une autre conductivité électrique qui est mesurée par un autre moyen de détection d'un autre moyen de mesure de concentration (20, 23, 27, 120, 123, 127) qui est disposé dans le conduit de liquide A (3, 103) ou dans le conduit d'alimentation en eau (2, 102) en aval du raccordement du conduit de liquide A (3, 103) sur le conduit d'alimentation en eau (2, 102).

8. Dispositif de préparation de liquide de dialyse (1, 101) comportant :
un conduit d'alimentation en eau (2, 102) destiné à l'alimentation en eau,
une pompe d'alimentation en eau qui est fournie sur le conduit d'alimentation en eau (2, 102),
un conduit de liquide A (3, 103) destiné à amener un liquide A qui contient du chlorure de sodium comme ingrédient principal conjointement avec au moins un autre ingrédient, le conduit de liquide A (3, 103) étant raccordé au conduit d'alimentation en eau (2, 102),
une pompe de liquide A (16, 116) qui est fournie sur le conduit de liquide A (3, 103),
un conduit de liquide B (4, 104) destiné à amener un liquide B qui comprend une solution aqueuse de bicarbonate de sodium, le conduit de liquide B (4, 104) étant raccordé au conduit d'alimentation en eau (2, 102),
une pompe de liquide B (18, 118) qui est fournie sur le conduit de liquide B (4, 104),
des moyens de mesure de concentration (20, 23, 27, 120, 123, 127), qui sont équipés d'une unité de détection (20a, 23a, 27a, 120a, 123a, 127a) en aval des positions de raccordement du conduit de liquide A (3, 103) sur le conduit d'alimentation en eau (2, 102), et
des moyens de commande (6, 106) destinés à commander le fonctionnement des pompes, le moyen de commande (6, 106) du dispositif de préparation de liquide de dialyse (1, 101) étant conçu pour commander un mélange de l'eau, du liquide A et du liquide B dans des proportions spécifiées afin de préparer un liquide de dialyse, et le moyen de commande (6, 106) étant équipé d'une unité de diagnostic (6a, 106a), **caractérisé en ce que**
l'unité de diagnostic (6a, 106a) est conçue pour diagnostiquer la précision du moyen de mesure de concentration (20, 23, 27, 120, 123, 127) par actionnement de la pompe de liquide A (16, 116) afin d'alimenter le conduit d'alimentation en eau (2, 102) uniquement en liquide A sans alimenter la pompe de liquide B (18, 118), l'unité de diagnostic (6a, 106a) étant conçue pour diagnostiquer la précision du moyen de mesure de concentration (20, 23, 27, 120, 123, 127) par comparaison d'une conductivité électrique du liquide A, dilué ou non dilué, qui est mesurée par l'unité de détection (20a, 23a, 27a, 120a, 123a, 127a) du moyen de mesure de concentration (20, 23, 27, 120, 123, 127), à une valeur préalablement réglée.

9. Dispositif de préparation de liquide de dialyse (1, 101) selon la revendication 8, lequel est en outre conçu pour
mesurer, lors de la mesure de la conductivité électrique du liquide A, un temps après que la pompe de liquide A (16, 116) a commencé à amener le liquide A et avant que le moyen de mesure de concentration (20, 23, 27, 120, 123, 127) ne mesure la concentration du liquide A, et
diagnostiquer ainsi une précision d'alimentation en liquide de la pompe de liquide B (18, 118).

10. Dispositif de préparation de liquide de dialyse (1, 101) selon l'une des revendications 8 et 9, lequel est en outre conçu, afin de préparer le liquide A dilué (3, 103), pour mélanger l'eau et le liquide A pour former un liquide de préparation du liquide A par introduction de l'eau dans le conduit d'alimentation en eau (2, 102).

11. Dispositif de préparation de liquide de dialyse (1, 101) selon l'une des revendications 8 à 10, comprenant en outre un afficheur, et conçu pour afficher (324) sur l'afficheur, lorsqu'un écart entre la conductivité électrique mesurée et la valeur préalablement réglée dépasse une valeur seuil spécifiée, un message indiquant que l'utilisateur doit vérifier si un type de liquide A, qui est utilisé comme paramètre d'entrée pour déterminer la valeur préalablement réglée correspond à un type de liquide A du liquide A qui est relié au conduit de liquide A (3, 103) .

12. Dispositif de préparation de liquide de dialyse (1, 101) selon la revendication 11, conçu en outre pour afficher sur l'afficheur (322) un message d'erreur indiquant un dysfonctionnement du dispositif de préparation de liquide de dialyse (1, 101) lorsqu'un type de liquide A, qui est utilisé comme un paramètre d'entrée pour déterminer la valeur préalablement réglée, correspond à un type de liquide A du liquide A qui est relié au conduit de liquide A (3, 103).

13. Dispositif de préparation de liquide de dialyse (1, 101) selon la revendication 12, comprenant un moyen d'interaction utilisateur et conçu en outre pour confirmer (326), lors de l'actionnement du moyen d'interaction utilisateur, que le type de liquide A du liquide A qui est utilisé comme paramètre d'entrée pour déterminer la valeur préalablement réglée, correspond au type de liquide A du liquide A qui est relié au conduit de liquide A (3, 103) afin d'afficher (322) sur l'afficheur du dispositif de préparation de liquide de dialyse un message d'erreur indiquant un dysfonctionnement du dispositif de préparation de liquide de dialyse.

14. Dispositif de préparation de liquide de dialyse (1, 101) selon l'une des revendications 8 à 13, comprenant en outre
une autre unité de détection d'un autre moyen de mesure de concentration (20, 23, 27, 120, 123, 127) qui est disposé dans le conduit de liquide A (3, 103) ou dans le conduit d'alimentation en eau (2, 102) en aval du raccordement du conduit de liquide A (3, 103) sur le conduit d'alimentation d'eau (2, 102), le dispositif de préparation de liquide de dialyse (1, 101) étant configuré pour utiliser comme valeur préalablement réglée une valeur basée sur l'autre conductivité électrique qui est mesurée par l'autre moyen de détection.
